# EUROPEAN PATENT APPLICATION

(11) **EP 4 702 929 A1**
(43) Date of publication of application: **04.03.2026**
(21) Application number: 25197936.5
(22) Date of filing: 25.08.2025
(51) Int. Cl.: A61B 6/03, A61B 6/46, A61B 6/51, A61B 6/00, A61B 6/50

(54) **IMAGE PROCESSING APPARATUS, IMAGE PROCESSING METHOD, AND PROGRAM**

(30) Priority: 26.08.2024 JP 2024144061
(71) Applicant: J. MORITA MANUFACTURING CORPORATION, Fushimi-ku, Kyoto-shi, Kyoto 6128533 (JP); Nihon University, Tokyo 102-8275 (JP)
(72) Inventor: ARAI, Yoshinori, Tokyo, 102-8275 (JP); NISHIMURA, Yu, Kyoto, 612-8533 (JP)
(74) Representative: Müller Hoffmann & Partner

(57) **Abstract**

An image processing apparatus includes a storage storing image data obtained by CT imaging of dentition constituent tissue, a processor, and a display. The processor generates three-dimensional image data of the dentition constituent tissue, identifies a segmentable region in the dentition constituent tissue, identifies a three-dimensional position of the segmentable region in a coordinate system of the three-dimensional image, generates a dentition developed image, identifies a segmentable region corresponding position corresponding to the three-dimensional position in the dentition developed image, and displays, on the display, the dentition developed image in which the segmentable region is displayed at the segmentable region corresponding position. By designation of the segmentable region corresponding position in the dentition developed image, on the basis of a correspondence between the segmentable region corresponding position and the three-dimensional position, a detail observation image of the segmentable region is generated, and a detail observation reference position that is a reference position for generating the detail observation image is determined according to a predetermined rule.

## Description

### BACKGROUND

### Technical Field

The present disclosure relates to a technology for making it easy to recognize a biological feature region.

### Description of the Background Art

Japanese Patent Application Laid-Open No. 2008-229322 discloses a technology of using X-ray projection data of a maxillofacial region obtained by X-ray computed tomography (CT) imaging to obtain a panoramic tomographic image of a dentition.

Japanese Translation of PCT International Application Publication No. 2021-528751 discloses a technology of displaying a detected region of interest on a CT scan image.

Japanese Patent Application Laid-Open No. 2021-174394 discloses a technology of performing lesion detection by inputting a CT image to a learned model.

Here, for example, it may be requested that a part of the panoramic tomographic image disclosed in Japanese Patent Application Laid-Open No. 2008-229322 be observed in detail. However, in the position designation using the panoramic tomographic image, it is difficult to designate an appropriate position in the buccolingual direction.

### SUMMARY

Therefore, an object of the present disclosure is to enable the position of the detail observation image to be appropriately set in the dentition developed image.

An image processing apparatus is an image processing apparatus that processes image data obtained by computed tomography (CT) imaging of dentition constituent tissue to generate an image for diagnosis, the image processing apparatus including: a storage that stores the image data; a processor; and a display that displays an observation image on the basis of output of the processor, wherein the processor generates three-dimensional image data of the dentition constituent tissue on the basis of the image data, identifies a segmentable region in the dentition constituent tissue on the basis of the image data, identifies a three-dimensional position at which the segmentable region is present in a coordinate system of the three-dimensional image, generates a dentition developed image in which the dentition constituent tissue is developed on the basis of the three-dimensional image data, identifies a segmentable region corresponding position corresponding to the three-dimensional position in the dentition developed image, displays, on the display, the dentition developed image in which the segmentable region is displayed at the segmentable region corresponding position, generates a detail observation image of the segmentable region from the three-dimensional image data on the basis of a correspondence between the segmentable region corresponding position and the three-dimensional position by designation of the segmentable region corresponding position in the dentition developed image, determines a detail observation reference position that is a reference position for generating the detail observation image according to a predetermined rule, and displays the detail observation image on the display.

An image processing method is an image processing method of processing image data obtained by computed tomography (CT) imaging of dentition constituent tissue to generate an image for diagnosis, the method including: generating three-dimensional image data of the dentition constituent tissue on the basis of the image data; identifying a segmentable region in the dentition constituent tissue on the basis of the image data; identifying a three-dimensional position at which the segmentable region is present in a coordinate system of the three-dimensional image; generating a dentition developed image in which the dentition constituent tissue is developed on the basis of the three-dimensional image data; identifying a segmentable region corresponding position corresponding to the three-dimensional position in the dentition developed image; displaying the dentition developed image in which the segmentable region is displayed at the segmentable region corresponding position; generating a detail observation image of the segmentable region from the three-dimensional image data on the basis of a correspondence between the segmentable region corresponding position and the three-dimensional position by designation of the segmentable region corresponding position in the dentition developed image; determining a detail observation reference position that is a reference position for generating the detail observation image according to a predetermined rule; and displaying the detail observation image.

A program is a program for processing image data obtained by computed tomography (CT) imaging of dentition constituent tissue to generate an image for diagnosis, the program being configured to cause a computer to execute processing of: generating three-dimensional image data of the dentition constituent tissue on the basis of the image data; identifying a segmentable region in the dentition constituent tissue on the basis of the image data; identifying a three-dimensional position at which the segmentable region is present in a coordinate system of the three-dimensional image; generating a dentition developed image in which the dentition constituent tissue is developed on the basis of the three-dimensional image data; identifying a segmentable region corresponding position corresponding to the three-dimensional position in the dentition developed image; displaying the dentition developed image in which the segmentable region is displayed at the segmentable region corresponding position; generating a detail observation image of the segmentable region from the three-dimensional image data on the basis of a correspondence between the segmentable region corresponding position and the three-dimensional position by designation of the segmentable region corresponding position in the dentition developed image; determining a detail observation reference position that is a reference position for generating the detail observation image according to a predetermined rule; and displaying the detail observation image.

According to the present disclosure, the position of the detail observation image can be appropriately set in the dentition developed image.

These and other objects, features, aspects and advantages of the present disclosure will become more apparent from the following detailed description of the present disclosure when taken in conjunction with the accompanying drawings.

### BRIEF DESCRIPTION OF THE DRAWINGS

FIG. 1 is a schematic diagram illustrating an image processing apparatus and a CT imaging apparatus according to a preferred embodiment;
FIG. 2 is a block diagram illustrating electrical configurations of the image processing apparatus and the CT imaging apparatus;
FIG. 3 is a functional block diagram of an arithmetic circuit;
FIG. 4 is a flowchart illustrating an example of processing in the image processing apparatus;
FIG. 5 is an explanatory diagram illustrating a field of view (FOV) region;
FIG. 6 is an explanatory diagram illustrating an example of a region in which dentition constituent tissue spreads;
FIG. 7 is an explanatory diagram illustrating positional relationships between the dentition constituent tissue and segmentable regions in the FOV region;
FIG. 8 is an explanatory diagram illustrating examples of coordinates of the segmentable regions in the dentition constituent tissue;
FIG. 9 is an explanatory diagram illustrating an example of processing of developing the dentition constituent tissue in a dentition developed image;
FIG. 10 is a diagram illustrating the dentition developed image;
FIG. 11 is a diagram illustrating a display example of the dentition developed image and a detail observation image;
FIG. 12 is a flowchart illustrating an example of processing of determining a detail observation reference position and an observation direction;
FIG. 13 is an explanatory diagram illustrating an example of designation of a segmentable region corresponding position using the dentition developed image;
FIG. 14 is an explanatory diagram illustrating an example of setting the detail observation image in a case where a root apex is designated;
FIG. 15 is an explanatory diagram illustrating an example of setting the detail observation image in a case where a root canal is designated;
FIG. 16 is a flowchart illustrating an example of another processing of determining the detail observation reference position and the observation direction;
FIG. 17 is an explanatory diagram illustrating an example of determining the observation direction using a dental arch curve;
FIG. 18 is an explanatory diagram illustrating an example of setting the detail observation image in a case where a tooth is designated;
FIG. 19 is an explanatory diagram illustrating an example of setting a reference position in a buccolingual direction using a horseshoe-shaped frame;
FIG. 20 is a flowchart illustrating a specific example of processing of step S10 in FIG. 4;
FIG. 21 is an explanatory diagram illustrating an example of moving operation processing of the detail observation image;
FIG. 22 is a diagram illustrating a dentition developed image according to a first modification;
FIG. 23 is an explanatory diagram illustrating a state in which a head is positioned by a head holder;
FIG. 24 is an explanatory diagram illustrating a positional relationship between the head holder and the dentition constituent tissue;
FIG. 25 is a schematic diagram illustrating an image processing apparatus according to a modification; and
FIG. 26 is a block diagram illustrating an electrical configuration of the image processing apparatus according to the modification.

### DESCRIPTION OF THE PREFERRED EMBODIMENTS

### {Preferred embodiment}

Hereinafter, an image processing apparatus, an image processing method, and a program according to a preferred embodiment will be described.

### <Configuration including image processing apparatus and CT imaging apparatus>

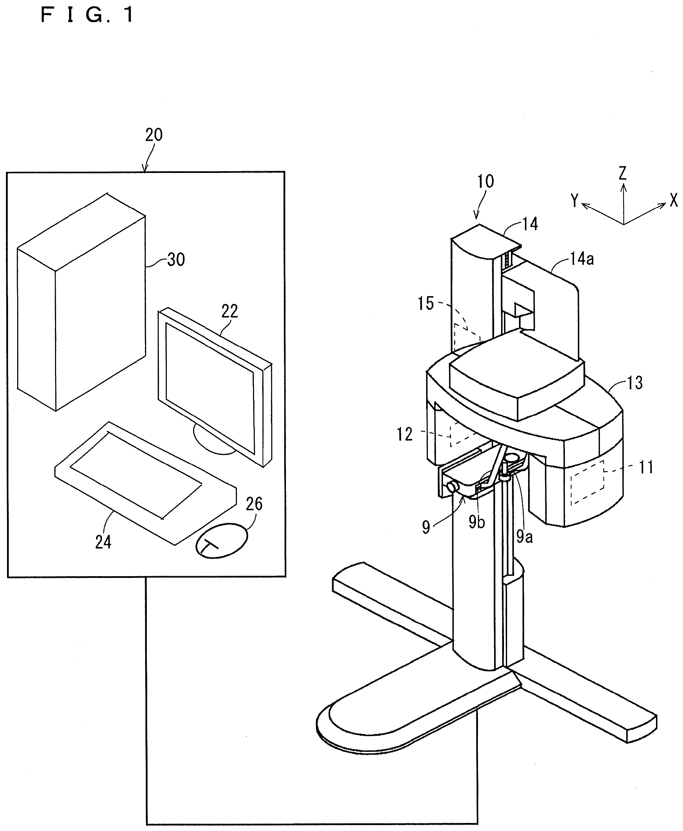

FIG. 1 is a schematic diagram illustrating an image processing apparatus 20 connected to a CT imaging apparatus 10.

The CT imaging apparatus 10 is an apparatus that obtains image data by performing computed tomography (CT) imaging of dentition constituent tissue. The obtained image data includes data on the dentition constituent tissue. The image processing apparatus 20 processes the image data to generate an image for diagnosis.

For example, the CT imaging apparatus 10 includes an X-ray generator 11, an X-ray detector 12, a turning arm 13, a support pillar 14, and an imaging processing unit 15. In a space where the CT imaging apparatus 10 exists, three-dimensional coordinates are set for arithmetic operation. As the three-dimensional coordinates, using, for example, the orientation of a subject positioned for imaging as a reference, a Z direction along the body axis direction, an X direction orthogonal to the Z direction and along the right-left direction of the subject, and a Y direction orthogonal to the Z direction and the X direction and along the front-back direction of the subject are set. In the present preferred embodiment, since the subject is positioned at a test position in the standing position, the Z direction is a direction perpendicular to the floor surface from which the support pillar 14 extends.

The X-ray generator 11 includes an X-ray tube and is configured to be able to emit an X-ray beam toward the subject. The X-ray detector 12 includes an X-ray detection sensor. The X-ray emitted from the X-ray generator 11 passes through the subject and is detected by the X-ray detector 12.

The turning arm 13 is, for example, a member formed in a U-shape opening downward. The X-ray generator 11 and the X-ray detector 12 are supported at both ends of the turning arm 13 while facing each other. The subject may be placed between the X-ray generator 11 and the X-ray detector 12. The subject is a human body part including the dentition constituent tissue, that is, a head including a jaw.

The support pillar 14 is erected so as to extend along the gravity direction (vertical direction). A cantilever arm 14a is supported by this support pillar 14 so as to be movable up and down. The turning arm 13 is turnably supported by the cantilever arm 14a. In accordance with a height position of the head, a height position of the turning arm 13 is adjusted along the support pillar 14.

The turning arm 13 is rotated with the head of the subject positioned between both ends of the turning arm 13. As a result, the X-ray generator 11 and the X-ray detector 12 rotate around the head. Consequently, the X-ray CT imaging on the head is performed, and the image data is obtained.

For example, when the turning arm 13 turns, the X-ray imaging is performed for each minute turning angle. As a result, the X-ray projection image data (frame data) for each minute turning angle is obtained. Three-dimensional volume data of the subject is generated by being based on an X-ray projection image data group (frame data group) subjected to imaging at different turning angles. This three-dimensional volume data is three-dimensional image data indicating the distribution of the X-ray absorption rate of the subject in the three-dimensional coordinate system.

The CT imaging apparatus 10 may include a head holder 9. The head holder 9 is a part that holds a head that is an imaging target. The head holder 9 may include a chin rest 9a that supports the chin. The chin rest 9a can support the front part and the lower part of the jaw of the head. As a result, the head is positioned at a fixed position in the front-back direction and the up-down direction. The head holder 9 may include a both-sides holder 9b that positions the head from both sides. The both-sides holder 9b may be, for example, an ear rod in contact with both ears of the head. The head is positioned at a fixed position in the right-left direction by the both-sides holder 9b.

The image processing apparatus 20 processes the image data obtained by CT imaging to generate an image for diagnosis. The image data obtained by the CT imaging may be an X-ray projection image data group for each minute turning angle or may be three-dimensional volume data. In the present preferred embodiment, an example in which the image data obtained by the CT imaging is an X-ray projection data group for each minute turning angle will be described.

The image processing apparatus 20 includes the processing unit 30, the display 22, and the user interface 24, 26.

The processing unit 30 is configured by a computer, a workstation, or the like. The processing unit 30 is connected to the imaging processing unit 15 of the CT imaging apparatus 10 in a wired or wireless manner, and can transmit and receive various data to and from the imaging processing unit 15. In the present preferred embodiment, the processing unit 30 can receive image data obtained by CT imaging from the imaging processing unit 15. Some or all of the functions of the processing unit 30 may be implemented by a cloud server.

The display 22 is, for example, a liquid crystal display, an organic electroluminescence (EL) display, or the like, and is connected to the processing unit 30 in a wired or wireless manner. The display 22 can display an observation image for diagnosis on the basis of the output for display of the processing unit 30.

The user interface 24, 26 is an apparatus that receives instructions from the user of the image processing apparatus 20. The user interface 24 may be, for example, a switch device such as a keyboard. The user interface 26 may be, for example, a pointer device such as a mouse. When the user interface is a switch device, a user's instruction can be input by the switch device alone. When the user interface is a pointer device, a user's instruction can be input by an operation on texts or an image displayed on the display 22. The user interface may be a touch panel.

### <Electrical configurations of image processing apparatus and CT imaging apparatus>

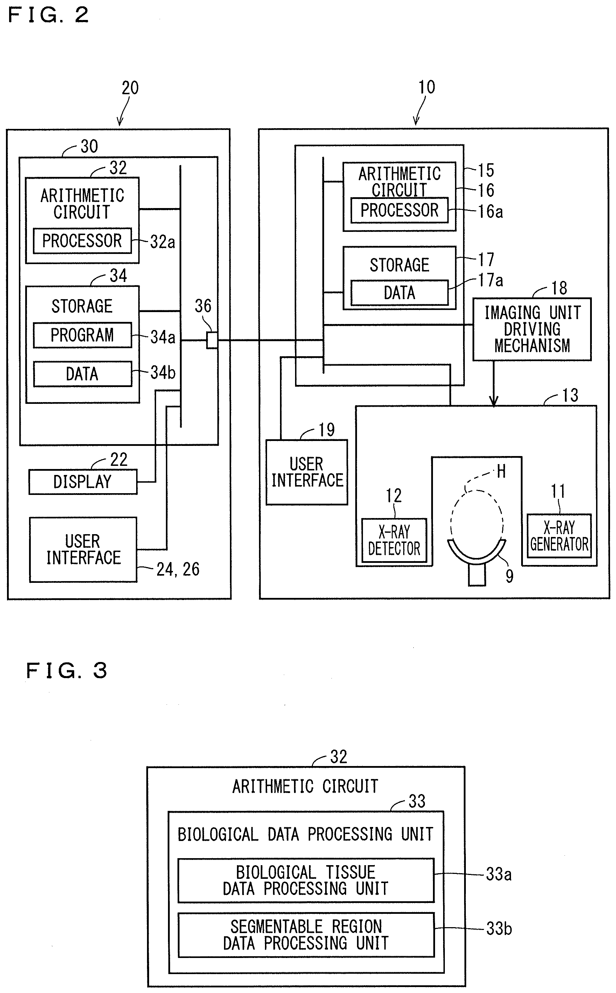

FIG. 2 is a block diagram illustrating electrical configurations of the image processing apparatus 20 and the CT imaging apparatus 10.

The CT imaging apparatus 10 includes the imaging processing unit 15, an imaging unit driving mechanism 18, and a user interface 19.

The imaging processing unit 15 is configured by a computer including an arithmetic circuit 16 and a storage 17.

The arithmetic circuit 16 includes a processor 16a. The processor 16a may be a central processing unit (CPU). The processor 16a may include a graphics processing unit (GPU).

The storage 17 is configured by a non-volatile storage such as a flash memory or a hard disk device. The storage 17 may be a storage circuit. The storage 17 stores a program. In the program, a procedure for the CT imaging apparatus 10 to perform the CT imaging is described.

The imaging unit driving mechanism 18 is connected to the imaging processing unit 15. The imaging unit driving mechanism 18 includes a turning driving mechanism for turning the turning arm 13. The turning driving mechanism includes an actuator and a transmission mechanism. The actuator is an electric motor or the like that generates a turning driving force. The transmission mechanism is a gear, a pulley, or the like that transmits rotational driving force of the actuator. The rotational driving force of the actuator is transmitted to the turning arm 13 via the transmission mechanism, and the turning arm 13 turns at a timing and a turning speed according to a command from the imaging processing unit 15.

The user interface 19 is an interface for giving an instruction to the CT imaging apparatus 10, and is a switch device, a pointer device, or the like. The user interface 19 is connected to the imaging processing unit 15. The user can provide various instructions to the CT imaging apparatus 10 through the user interface 19.

The X-ray generator 11 and the X-ray detector 12 are also connected to the imaging processing unit 15. The X-ray generator 11 emits an X-ray at timing and output according to a command from the imaging processing unit 15, and the X-ray detector 12 outputs a detection result to the imaging processing unit 15.

The processor 16a executes processing according to the program in the storage 17, so that the imaging unit driving mechanism 18 controls the turning operation of the turning arm 13 and controls the X-ray emission operation of the X-ray generator 11. The detection result of the X-ray detector 12 is input to the imaging processing unit 15 for each minute turning angle of the turning arm 13, and the processor 16a generates the X-ray projection image data for each minute turning angle on the basis of the detection result. The data is stored as data 17a of the X-ray projection image data group for each minute turning angle in the storage 17.

The image processing apparatus 20 includes the processing unit 30, the display 22, and the user interface 24, 26.

The processing unit 30 is connected to the display 22 and the user interface 24, 26. The processing unit 30 may receive instructions from a user via the user interface 24, 26. The processing unit 30 can control display of the display 22.

The processing unit 30 is configured by a computer including an arithmetic circuit 32 as a processor and a storage 34.

The arithmetic circuit 32 includes a processor 32a. The processor 32a may be a central processing unit (CPU). The processor 32a may include a processor for a graphics processing unit (GPU) or artificial intelligence (AI).

The storage 34 is configured by a non-volatile storage such as a flash memory or a hard disk device. The storage 34 may be a storage circuit. The storage 34 stores a program 34a and data 34b.

In the program 34a, a procedure for the image processing apparatus 20 to process image data obtained by CT imaging to generate an image for diagnosis is described.

The data 34b includes image data transmitted from the CT imaging apparatus 10, data generated by processing for generating the above-described image for diagnosis, and the like. The data 34b may be left as history data or may be erased after processing.

The processing unit 30 includes a connection port 36. The processing unit 30 is connected to the CT imaging apparatus 10 through the connection port 36. The connection port 36 may include a terminal connected to a signal line of a wired cable extending from the CT imaging apparatus 10 and a circuit for communication processing. The processing unit 30 and the CT imaging apparatus 10 may be wirelessly connected, and in this case, the connection port 36 may include a circuit for wireless processing. The data 17a of the X-ray projection image data group of the CT imaging apparatus 10 is transmitted to the processing unit 30 through the connection port 36 and stored in the storage 34.

The arithmetic circuit 32 reads the program 34a stored in the storage 34 and executes the processing described in the program 34a, so that the arithmetic circuit 32 can execute various types of processing of generating an image for diagnosis as described later. The processing unit 30 may control the CT imaging performed by the CT imaging apparatus 10.

As illustrated in FIG. 3, the processing functional unit achieved by the arithmetic circuit 32 reading the program 34a may include a biological data processing unit 33. The biological data processing unit 33 may include a biological tissue data processing unit 33a and a segmentable region data processing unit 33b.

The processing performed by the biological tissue data processing unit 33a is processing of generating three-dimensional data of the dentition constituent tissue on the basis of the image data. The dentition constituent tissue is, for example, tissue including a dentition and an alveolar bone. The dentition constituent tissue may be tissue including a dentition and a jawbone. The jawbone in the dentition constituent tissue may be a jawbone in a region supporting teeth, and may include not only the alveolar bone but also a peripheral region thereof. In the present application, the alveolar bone is a partial region of the jawbone, and is understood as a part of the jawbone that constitutes the alveolars and supports the teeth. This understanding is in line with the description of the Dental Medicine Dictionary. The dentition constituent tissue may have a horseshoe shape in plan view. Note that the plan view may be considered as a plan view in which a direction along a direction from the head side to the leg side in the axial direction of the body axis is a line-of-sight direction. The dentition constituent tissue may spread so as to have a thickness in the buccolingual direction. The dentition constituent tissue may be, for example, tissue having at least a thickness of the dentition in the buccolingual direction. The dentition constituent tissue may be tissue having a thickness of a region supporting the teeth of the jawbone. The dentition constituent tissue may include upper and lower dental arches and upper and lower jawbones supporting the upper and lower dental arches. Each of the upper and lower dental arches includes a plurality of teeth arranged in an arch shape. The upper jawbone has an alveolar bone that supports the teeth of the upper dental arch. The lower jawbone has an alveolar bone that supports the teeth of the lower dental arch.

For example, the biological tissue data processing unit 33a identifies respective regions of the plurality of teeth and respective regions of the upper and lower jawbones in the three-dimensional volume data. The identification of the regions of the teeth and the upper and lower jawbones may be performed by applying a learned machine learning model. For example, as training data, a large number of pieces of data in which regions of teeth and upper and lower jawbones are mapped to the three-dimensional volume data are prepared. A machine learning model learned to segment the tooth regions and the upper and lower jawbone regions in the three-dimensional volume data is prepared using the training data. The machine learning model may be, for example, a model learned on the basis of a semantic segmentation algorithm. By applying the learned machine learning model to the three-dimensional volume data, respective regions of the plurality of teeth and respective regions of the upper and lower jawbones in the three-dimensional coordinate system are identified. As a result, the dentition region and the alveolar bone region are differentiated from each other on the basis of the three-dimensional volume data.

The identification of respective regions of the plurality of teeth and respective regions of the upper and lower jawbones in the three-dimensional volume data may be performed by region extraction processing, for example, pattern matching processing, which is regulated as a rule in advance.

The segmentable region data processing unit 33b is processing of identifying the segmentable region in the dentition constituent tissue on the basis of the image data. The segmentable region in the dentition constituent tissue is a portion that can be segmented from other portions in the dentition constituent tissue on the basis of the image data obtained by the CT imaging. For example, the segmentable region is a portion that is segmented on the basis of the presence or absence of an actual object in the dentition constituent tissue, a difference in X-ray absorption rate, or the like. The segmentable region may be a wide area or a local portion.

The segmentable region may be, for example, an anatomical differentiation region. The anatomical differentiation region is a region that anatomically differentiates the dentition constituent tissue. More specifically, the anatomical differentiation region may be each of a plurality of teeth, or may be each of upper and lower jawbones. The anatomical differentiation region may be a root canal or a root apex of each of the plurality of teeth. The anatomical differentiation region may be a mandibular canal.

In a case where the segmentable region is the anatomical differentiation region, a part or all of the processing performed by the segmentable region data processing unit 33b may be performed by the biological tissue data processing unit 33a. For example, identification of the plurality of teeth and jawbones may be performed by the biological tissue data processing unit 33a, and the processing of identifying the root canal, the root apex, or the mandibular canal in the tooth or jawbone may be performed by the segmentable region data processing unit 33b. That is, in a case where the segmentable region includes teeth and jawbones, it may be considered that the biological tissue data processing unit 33a generates three-dimensional image data of the dentition constituent tissue on the basis of the image data and simultaneously identifies the segmentable region in the dentition constituent tissue for the teeth and jawbones.

The segmentable region may be, for example, a biological feature region in the dentition constituent tissue. The biological feature region is, for example, a region in which a lesion has occurred in the dentition constituent tissue. The lesion is a change caused by disease. The region where the lesion has occurred shows a distribution of the X-ray absorption rate different from the distribution of the X-ray absorption rate shown by the normal dentition constituent tissue. The disease is, for example, chronic pyogenic apical periodontitis, tooth root granuloma, and the like. In the case of apical periodontitis, a site having a lower X-ray absorption rate than that in the normal state and in its surrounding is generated at the peripheral edge of the tip of the tooth root. Therefore, in a case where the low X-ray absorption rate region spreads in the peripheral edge portion of the tooth root, apical periodontitis can be identified. For other lesions, a region where a lesion has occurred can be identified on the basis of three-dimensional volume data obtained by CT imaging on the basis of a position in the dentition constituent tissue such as a tooth or a jawbone, a spread pattern or a distribution pattern of an X-ray absorption rate region, and the like. Here, in a case where the segmentable regions are, for example, a plurality of lesion regions, naturally, the assembly of the segmentable regions is constituted by collecting individual lesion regions. As this individual lesion region, an individual segmentable region constituting the assembly of segmentable regions may be referred to as unital segmentable region. A region where a plurality of unital segmentable regions are collected may be referred to as collective segmentable region. The image of the segmentable region may be referred to as segmentable region image.

The identification of the segmentable region in the segmentable region data processing unit 33b may be performed by applying a learned machine learning model similar to that in the identification of the dentition constituent tissue. For example, as training data, a large number of pieces of data in which regions of segmentable regions are mapped to the three-dimensional volume data are prepared. A machine learning model learned to segment the regions of segmentable regions in the three-dimensional volume data is prepared using the training data. By applying the learned machine learning model to the three-dimensional volume data, segmentable regions in the three-dimensional coordinate system are identified.

The identification of the segmentable region in the three-dimensional volume data may be performed by image extraction processing, for example, pattern matching processing.

The identification of the segmentable region may not be performed on the basis of the three-dimensional volume data. For example, the segmentable region may be identified by applying a machine learning model, pattern matching, or the like on the basis of slice data sliced in any direction of three-dimensional volume data.

In the present preferred embodiment, an example in which the segmentable region is the anatomical differentiation region included in the dentition constituent tissue will be mainly described.

### <Example of processing in processing unit>

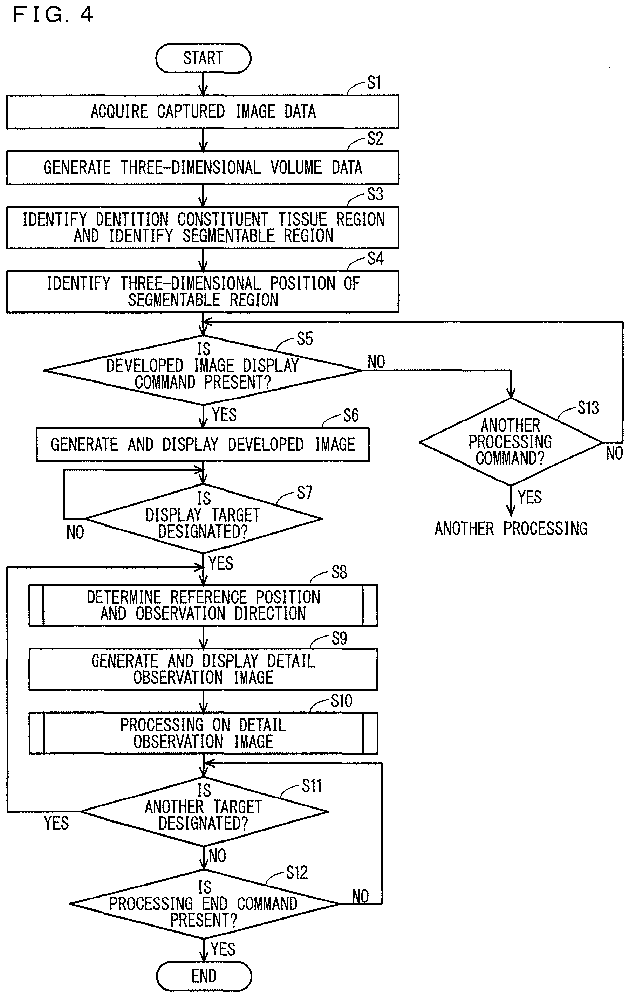

An example of processing by the processing unit 30 will be described with reference to the flowchart of FIG. 4.

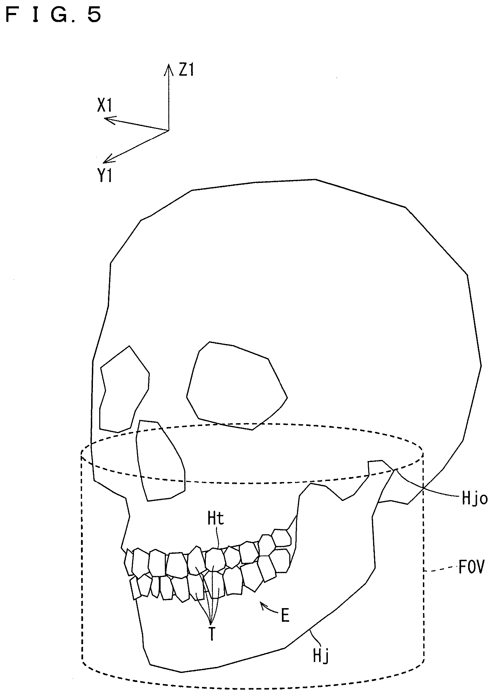

After starting the processing, in step S1, the processing unit 30 acquires the captured image data from the CT imaging apparatus 10. The captured image data is, for example, data of a field of view (FOV) region including a dental arch Ht and a jawbone Hj in a head H (See FIG. 5). For example, the FOV may be set in a cylindrical region, an elliptic columnar region, or a triangular columnar region.

In the next step S2, the processing unit 30 generates three-dimensional volume data on the basis of the captured image data.

In the present preferred embodiment, in step S1, the captured image data acquired from the CT imaging apparatus 10 is an X-ray projection image data group. Furthermore, in step S2, three-dimensional volume data is generated on the basis of the X-ray projection image data group. In the CT imaging apparatus 10, the three-dimensional volume data may be generated on the basis of the X-ray projection image data group. In this case, the processing unit 30 may acquire the three-dimensional volume data as image data.

It is also conceivable that the CT imaging apparatus 10 generates a plurality of tomographic images on the basis of the X-ray projection image data group. In this case, the processing unit 30 may generate the three-dimensional volume data by acquiring a plurality of tomographic images as the image data from the CT imaging apparatus 10 and superimposing the plurality of tomographic images.

In next step S3, the processing unit 30 executes processing of generating three-dimensional image data of the dentition constituent tissue on the basis of the image data and processing of identifying a segmentable region L in the dentition constituent tissue on the basis of the image data.

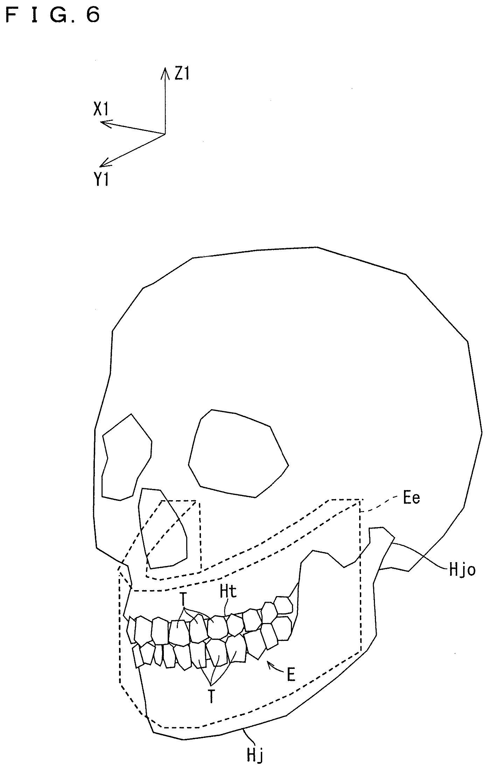

Note that, as illustrated in FIG. 6, a region Ee in which dentition constituent tissue E spreads includes the dental arch Ht in which a plurality of teeth T are arranged and the upper and lower jawbones Hj as described above. The dentition constituent tissue may include a temporomandibular joint Hjo. In the three-dimensional volume data, the region Ee in which the dentition constituent tissue E spreads may be referred to as dentition constituent tissue region Ee. The dentition constituent tissue region Ee is a horseshoe-shaped region set for arithmetic operation, and may be any region that conforms to the shape and position of the dentition constituent tissue. A horseshoe-shaped frame F to be described later is an example of the dentition constituent tissue region Ee. The shape of the dentition constituent tissue region Ee may conform to the shape of the dentition constituent tissue E having the standard skeleton, and when the shape of the dentition constituent tissue E of the individual is known, the shape may be set to a shape conforming to that individual.

As described above, the processing of generating the three-dimensional image data of the dentition constituent tissue on the basis of the image data may be processing of identifying respective regions of the plurality of teeth and respective regions of the upper and lower jawbones on the basis of the three-dimensional volume data, combining the respective regions, and generating the three-dimensional data of the dentition constituent tissue.

Respective regions of the plurality of teeth and respective regions of the upper and lower jawbones may be identified by the surface layers of the teeth and jawbones, i.e. the boundaries between the interior and the exterior of the teeth and the jawbones. The plane constituted by the boundary may be considered as a surface. That is, respective regions of the plurality of teeth and respective regions of the upper and lower jawbones may be identified as the surface shape of each tooth and jawbone in the three-dimensional coordinate system of the three-dimensional volume data. Tissues of different functions constituting the living body, such as teeth and jawbones, may be referred to as functional dentition constituent tissue. For example, teeth are functional dentition constituent tissue having a function of biting food, and jawbones are functional dentition constituent tissue having a function of supporting teeth. The dentition constituent tissue may be considered to include a plurality of functional dentition constituent tissues. Since the region facing the teeth in the boundary of the jawbone is important, a learning model including segmentation of the alveolar inner wall may be prepared so that the alveolar inner wall can be detected with high accuracy.

In addition, the processing of identifying the segmentable region on the basis of the image data may be processing of identifying the segmentable region in the dentition constituent tissue on the basis of the three-dimensional volume data as described above. In the present preferred embodiment, an example in which the segmentable region is the anatomical differentiation region will be described.

The segmentable regions may be identified by the surface layers of the teeth and jawbones, i.e. the boundaries between the interior and the exterior of the teeth and the jawbones similarly to the respective regions of the teeth and jawbones. The plane constituted by the boundary may be considered as a surface. It is conceivable that the segmentable region is an elongated portion such as a root canal or an extremely small portion such as a root apex, and thus the segmentable region may be identified by a straight line, a curve, or a point in three-dimensional coordinates. The identification of the segmentable region may be considered as differentiation between a region that is the segmentable region and a region that is not the segmentable region.

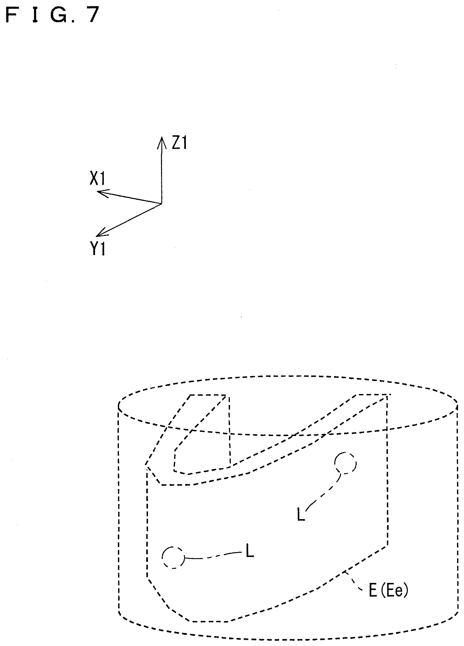

By step S3, three-dimensional image data of the dentition constituent tissue E including the dentition and the jawbone is generated in the FOV, and the segmentable region L in the dentition constituent tissue E is identified (See FIG. 7). FIG. 7 illustrates some segmentable regions L.

Note that, in the present preferred embodiment, description will be made on the premise that some kind of segmentable region is present. In a case where no segmentable region is identified, the processing may end.

In step S4, the processing unit 30 calculates a three-dimensional position where the identified segmentable region is present in the coordinate system of the three-dimensional image data of the dentition constituent tissue E. As the three-dimensional coordinates of the three-dimensional image data, in arithmetic operation, on the basis of the spatial coordinates of the CT imaging apparatus, for example, on the basis of the orientation of the subject at the time of imaging, an X1 direction which is the same direction as the X direction, a Y1 direction which is the same direction as the Y direction, and a Z1 direction which is the same direction as the Z direction are set. The three-dimensional position of the segmentable region may be coordinates itself identifying the segmentable region identified in step S3. For example, it may be a three-dimensional coordinates group indicating the surface of the segmentable region. The three-dimensional position where the segmentable region is present may be referred to as segmentable region three-dimensional position.

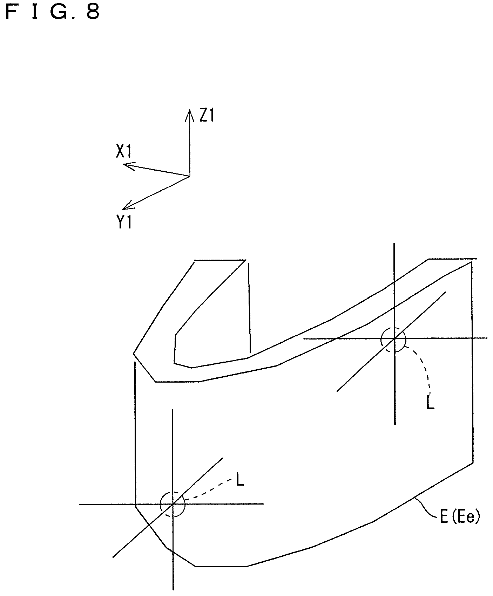

It is also conceivable that the three-dimensional position of the segmentable region is coordinates calculated on the basis of the segmentable region identified in step S3. For example, in a case where the segmentable region is a biological feature region, the three-dimensional position of the segmentable region may be point coordinates located within the boundary of the biological feature region. The coordinates of the biological feature region may be, for example, the geometric center of the surface of the lesion region detected in step S3 (See FIG. 8). It may be any position (for example, a lower end or an upper end) on the surface of the lesion region.

In next step S5, the processing unit 30 determines the presence or absence of the display command of the developed image. The display command of the developed image is received using the user interface 24, 26. When the user inputs the display command of the developed image using the user interface 24, 26, the processing unit 30 determines that the display command of the developed image is present, and the processing proceeds to step S6. In a case where the user does not input the display command of the developed image, the processing unit 30 determines that the display command of the developed image is absent, and the processing proceeds to step S13.

In step S13, the processing unit 30 determines the presence or absence of another command via the user interface 24, 26, and the like. When it is determined that another command is present, the processing unit 30 executes the corresponding other processing. When it is determined that another command is absent, the processing returns to step S5, and the processing unit 30 repeats the processing of determining the presence or absence of the display command of the developed image.

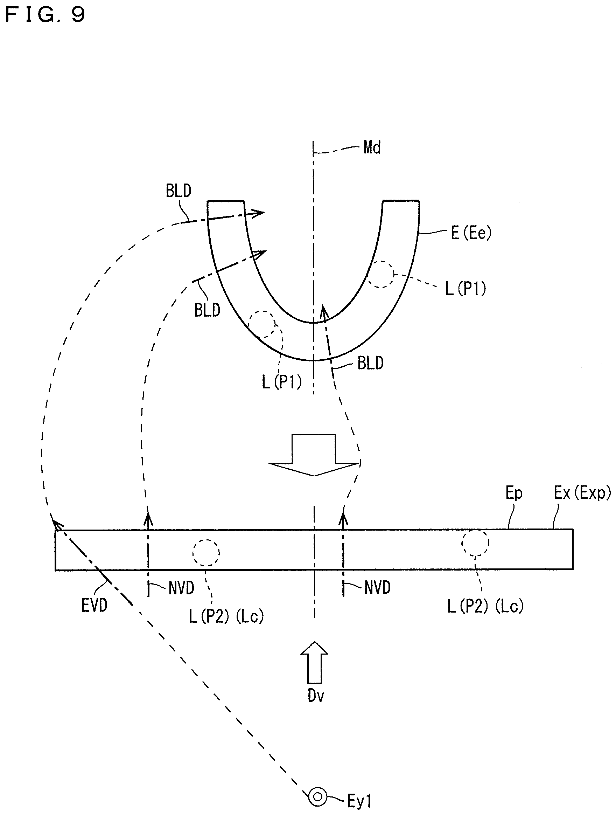

In a case where the processing proceeds to step S6, the processing unit 30 generates a dentition developed image Ep in which the dentition constituent tissue E is developed on the basis of the three-dimensional image data of the dentition constituent tissue E. The dentition developed image Ep is an image in which the dentition constituent tissue E having an arch shape or a horseshoe shape in plan view is developed so as to form a straight line in plan view (See FIG. 9). For example, the dentition developed image Ep is generated as follows. That is, at each coordinate position in the up-down direction, a curve having an arch shape or a horseshoe shape passing through the center in the buccolingual direction of the dentition constituent tissue E is calculated. At each position along the curve, the presence or absence or transmittance of an image of the dentition constituent tissue E in a direction orthogonal to a tangent of the curve is calculated. The presence or absence or transmittance of an image at respective coordinates on the curve is expressed as presence or absence or transmittance of an image at respective linear coordinates. At respective coordinate positions in the up-down direction, the above processing is performed and data of the processing is overlapped in the up-down direction, whereby the dentition developed image Ep is generated.

The generation of the dentition developed image Ep may be performed by development of the dentition constituent tissue E or by development of the region Ee, in which the dentition constituent tissue E spreads. The development used herein may be regarded as making a large curvature smaller in plan view. The curvature may be the curvature of a line passing through the center in the buccolingual direction of the dentition constituent tissue E or the region Ee. The development may be regarded as making such a large curvature smaller when seen in the Z1 direction. Making a large curvature smaller may include changing a curved state to a flat state, more specifically, may include changing the dentition constituent tissue E or the region Ee from a curved state to a flat state in plan view. A state with a small curvature may include a case in which the curvature is zero.

Note that, in a case where the three-dimensional image data of the dentition constituent tissue E is expressed by the translucent data of the surface of the dentition constituent tissue E, the boundary portion of the dentition constituent tissue in the dentition developed image Ep is expressed in a dark color with low transmittance.

The dentition developed image Ep is a kind of panoramic image in which the dental arch is planarly developed. The dentition developed image Ep may also extend to the alveolar bone. Furthermore, the dentition developed image Ep may also extend to the jawbone. The dentition developed image Ep may be an image in which the dentition constituent tissue E is passed through in the thickness direction thereof, or may be a tomographic image at an arbitrary position in the thickness direction.

The dentition developed image Ep is preferably an image in which the entire region of the dentition constituent tissue E is viewed frontally. As illustrated in FIG. 9, the image in which the entire region of the dentition constituent tissue E is viewed frontally is, assuming that the dentition developed image Ep is developed in the direction orthogonal to a median line Md of the head H, an image in which the entire dentition constituent tissue E is viewed from the front of the median line of the head H (from a frontally-viewing direction Dv in which the front teeth region is viewed frontally). The image data of the dentition developed image Ep has a thickness of the dentition constituent tissue E. The image data of the dentition developed image Ep has at least a thickness of dental arches in the buccolingual direction.

Not only the three-dimensional image data of the dentition constituent tissue E but also data obtained by converting the three-dimensional volume data into a panoramic image may be superimposed on the dentition developed image Ep. The dentition developed image Ep does not necessarily develop so as to form a linear shape in plan view, and may include curvature or bending. That is, the dentition developed image Ep may be an image in which the entire dentition constituent tissue E can be observed at a glance. For example, the development may be performed as processing such that the amount of forward displacement becomes larger in the regions on the right and left end sides of the dentition constituent tissue region Ee than in the region near the center. When linear development or substantially linear development in plan view is expressed as "being developed flatly", the dentition developed image Ep may be formed so as to become a flatly developed image. Image processing for generating the dentition developed image Ep so that the entire dentition constituent tissue E can be observed at a glance may be referred to as development processing. In particular, the development processing of flatly developing may be referred to as flat development processing. The development of flatly developing may be referred to as flat development.

By similarly applying the above processing to the segmentable region L, a segmentable region corresponding position P2 corresponding to the three-dimensional position P1 is calculated in the dentition developed image Ep. The segmentable region corresponding position P2 may be regarded as a position obtained by converting the three-dimensional position P1 of the segmentable region identified in step S4 by the same processing as the geometric conversion processing of developing the dentition constituent tissue E in the dentition developed image Ep. The three-dimensional position P1 can be determined by, for example, a coordinates group indicating the surface of the segmentable region or representative coordinates indicating the segmentable region. The representative coordinates of the segmentable region may be a center, for example, a geometric center or a centroid.

As the arithmetic operation target, the three-dimensional position P1 may be the position of a spot (that is, a position indicating a specific point), but, in a case where the segmentable region has a spread, may be the position of at least a part of the region. For example, it is conceivable that the three-dimensional position P1 is, for example, a certain region around the center including the center. The three-dimensional position P1 may be a position of the entire segmentable region. Therefore, the corresponding position P2 may also be a case of the position of the spot or a case of the position of the region as the arithmetic operation target.

A region set in arithmetic operation in which the dentition constituent tissue region Ee is developed may be considered as a dentition developed region Ex. Also for the dentition constituent tissue region Ee, the development of flatly developing may be referred to as flat development, and in particular, the dentition developed region Ex in which flat development is performed may be considered as a dentition flatly-developed region Exp.

The development of the dentition constituent tissue E may be performed by development processing of developing the image data of the dentition constituent tissue E in the dentition constituent tissue region Ee so as to conform to the shape of the dentition developed region Ex. Hereinafter, it will be described that the region occupied by the dentition constituent tissue E and the dentition constituent tissue region Ee coincide with each other, and the region occupied by the dentition developed image Ep and the dentition developed region Ex coincide with each other.

The state of the dentition constituent tissue region Ee before development may be referred to as pre-development curved state, and the state of the dentition developed region Ex after development may be referred to as post-development at-a-glance state. It is preferable that the development processing is performed such that a buccolingual direction BLD in the pre-development curved state corresponds to a frontally-viewing direction NVD in the post-development at-a-glance state.

The frontally-viewing direction may be a direction orthogonal to the frontally-viewed surface of the dentition developed region Ex or a normal direction, but a line-of-sight direction based on another idea may be set. For example, a virtual viewpoint Ey1 may be set on the center of the dentition developed region Ex in the frontally-viewing line-of-sight direction to perform image processing along a line-of-sight direction EVD from this viewpoint.

In the dentition developed region Ex and/or the dentition developed image Ep, a region existing at the segmentable region corresponding position P2 and corresponding to the segmentable region L may be referred to as segmentable region corresponding region Lc.

Since the dentition constituent tissue region Ee has a thickness in the buccolingual direction, the dentition developed region Ex also has a thickness in the frontally-viewing direction corresponding to the thickness in the buccolingual direction. Furthermore, since the dentition constituent tissue E has the above-described thickness in the buccolingual direction, the dentition developed image Ep also has a thickness in the frontally-viewing direction corresponding to the buccolingual direction.

Since the dentition developed region Ex has a thickness corresponding to the dentition constituent tissue region Ee, unlike the thin panoramic tomograph, even if there is a deviation in the buccolingual direction at the position where the segmentable region is present, the dentition developed region Ex can be accommodated in the region (in the image processing, the segmentable region image is accommodated in the region without blurring). The coordinates of the three-dimensional position P1 may be calculated as coordinates in the FOV region or as coordinates in the dentition constituent tissue region Ee.

In the development, arithmetic operation for associating the three-dimensional position P1 of the segmentable region with the segmentable region corresponding position P2 may be performed. The arithmetic operation for performing this association may be referred to as original coordinates arithmetic operation. In the original coordinates arithmetic operation, arithmetic operation of identifying which position in the buccolingual direction the three-dimensional position P1 of the segmentable region is located may be performed. In addition, arithmetic operation of identifying which position in the frontally-viewing direction corresponding to the buccolingual direction the segmentable region corresponding position P2 is located may be performed.

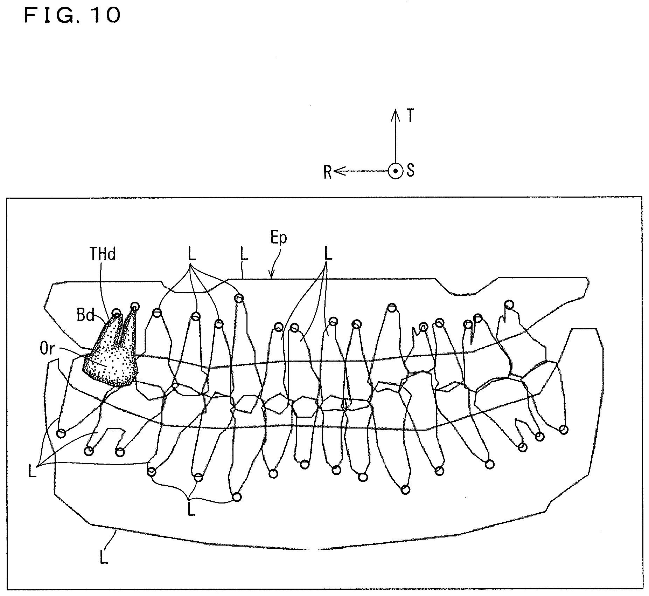

Then, as illustrated in FIG. 10, the processing unit 30 displays, on the display 22, the dentition developed image Ep in which the segmentable region L is displayed at the segmentable region corresponding position.

For arithmetic operation, three-dimensional coordinates may be set in the dentition developed region Ex. For example, the right-left extending direction due to the development of the dentition developed region Ex is defined as an R direction. A direction parallel to the body axis is defined as a T direction. In the present preferred embodiment, the T direction, the Z direction, and the Z1 direction are the same direction. A direction parallel to the frontally-viewing direction is defined as an S direction. In the dentition developed image Ep, the right-left direction is defined as the R direction, and the up-down direction is defined as the T direction. When conditions for identifying RT coordinates on the dentition developed image Ep and for identifying the S direction are determined, mutual identification with X1Y1Z1 coordinates is enabled. Therefore, like the segmentable region corresponding region Lc in the dentition developed region Ex, the region in which S coordinates are determined together with the RT coordinates can be mutually identified with the X1Y1Z1 coordinates of the segmentable region L. The coordinates on the three-dimensional image data such as the X1Y1Z1 coordinates may be referred to as three-dimensional image data coordinates, and the three-dimensional coordinates may be referred to as three-dimensional image data three-dimensional coordinates. The coordinates on the dentition developed region Ex such as RST coordinates may be referred to as dentition developed region coordinates, and the three-dimensional coordinates may be referred to as dentition developed region three-dimensional coordinates.

The segmentable region L displayed in the dentition developed image Ep may be expressed by the boundary of the segmentable region L. For example, the boundaries of the teeth and the jawbone as the segmentable regions L may be indicated by lines. The segmentable region may be expressed by a line or a representative point. In a case where the segmentable region is an extremely small portion such as a root apex and is a portion that is difficult to be noticed, in order to indicate the segmentable region, brightness or color may be changed from that of other portions, or a characteristic mark may be displayed, or display in a blinking manner may be performed.

In the example illustrated in FIG. 10, the boundaries of the teeth and the jawbones as the segmentable regions L are indicated by lines, and the root apexes are indicated by a mark having a round shape. For example, as in a tooth image THd, translucent processing of the functional dentition constituent tissue may be performed such that the boundary of the functional dentition constituent tissue is constituted by uniform and sparse dots. As a result, a surface region Or orthogonal to the line-of-sight direction has high transparency, and a surface region Bd along the line-of-sight direction has low transparency, so that the boundary can be easily visually recognized while being translucent. Note that the dots may be non-uniform to such an extent that difficulty in visual recognition is not caused. Since it is sufficient that the boundary surface has transparency, the image processing is not limited to dots, and may be image processing of a see-through surface such as a mesh. In both the dot and the mesh, the sparseness and denseness vary depending on the line-of-sight direction, so that the transparency varies. Image processing in which the transparency changes depending on the line-of-sight direction may be referred to as line-of-sight direction-corresponding transparency image processing.

An element that increases transparency, such as dots in a sparse state as compared with a dense state or eliminated dots, is referred to as pro-transparency element. Furthermore, increasing the transparency is referred to as pro-transparency.

An element that lowers the transparency, such as dots in a dense state as compared with the sparse state or nonexistent state, is referred to as anti-transparency element. Furthermore, lowering the transparency is referred to as anti-transparency.

The line-of-sight direction-corresponding transparency image processing may be a combination of at least any one of the following group A and at least any one of the following group B.
Group A:
   - Processing of enlarging or increasing the pro-transparency element of the frontally-viewed boundary surface
   - Processing of promoting pro-transparency of the frontally-viewed boundary surface
   - Processing of downsizing, or reducing or eliminating the anti-transparency element of the frontally-viewed boundary surface
   - Processing of suppressing anti-transparency of the frontally-viewed boundary surface
Group B:
   - Processing of downsizing, or reducing or eliminating the pro-transparency element of the obliquely-viewed or laterally-viewed boundary surface
   - Processing of suppressing pro-transparency of the obliquely-viewed or laterally-viewed boundary surface
   - Processing of enlarging or increasing the anti-transparency element of the obliquely-viewed or laterally-viewed boundary surface
   - Processing of promoting anti-transparency of the obliquely-viewed or laterally-viewed boundary surface

The line-of-sight direction-corresponding transparency image processing may also be performed on the alveolar bone and the jawbone part.

Step S5 may be omitted, and after the processing of step S4 is ended, the processing of step S6 may be performed regardless of the presence or absence of a command.

In next step S7, the processing unit 30 determines whether the display target is designated. The designation of the display target is designated by designating the segmentable region corresponding position in the dentition developed image Ep. The designation of the segmentable region corresponding position may be performed, for example, by selecting any one of the plurality of segmentable regions L with a switch such as a keyboard for selection or a pointer device such as a mouse. The designation of the segmentable region corresponding position may be performed by designating a position in any one of the segmentable regions with a pointer device such as a mouse. The processing of step S7 is repeated until the display target is designated, and when the display target has been designated, the processing proceeds to next step S8.

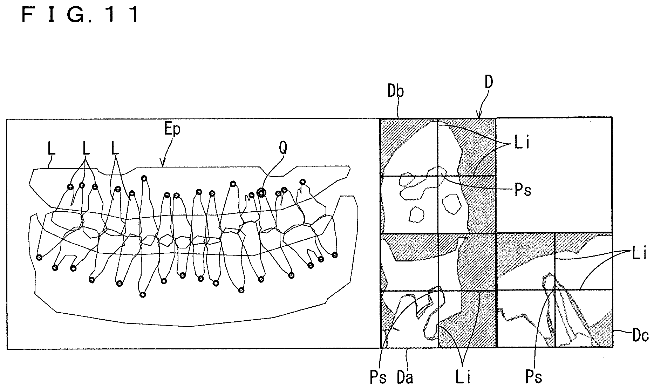

In steps S8 and S9, using the segmentable region corresponding position designated in step S7, the detail observation image D of the segmentable region is generated from the three-dimensional image data on the basis of the correspondence between the segmentable region corresponding position and the three-dimensional position (segmentable region three-dimensional position) and displayed. Then, the detail observation image D is displayed on the display 22 simultaneously with the dentition developed image Ep (See FIG. 11). In the dentition developed image Ep, an image Q indicating a designated position may be provided. In FIG. 11, a circular mark larger than the mark indicating the root apex is provided. The image Q indicating the designated position may be an image exhibiting a color, a shape, or a change that enables differentiation from the dentition developed image Ep spreading in the background. For example, the image Q may be an image exhibiting a hue different from that of the dentition developed image Ep. More specifically, the image Q may be an image exhibiting a warm color that is easily noticeable, for example, red, orange, or yellow. The image Q may be an image with a display change that enables differentiation from the dentition developed image Ep, for example, blinking, a color change, or a shape change. The image Q may have a shape that enables differentiation from the dentition developed image Ep, for example, a circle, a regular polygon, a radiation shape, a cross shape, an exclamation mark shape, or the like.

The detail observation image D is an image that can be observed in more detail than the dentition developed image Ep and is suitable for more detailed diagnosis. In the present preferred embodiment, an example in which the detail observation image D includes a plurality of cross-sectional images Da, Db, Dc, more specifically, three cross-sectional images Da, Db, Dc orthogonal to each other will be described (See FIG. 11).

The cross-sectional image is preferably a cross section passing through a detail observation reference position Ps suitable for detail observation of the segmentable region. The three cross-sectional images Da, Db, Dc may be cross sections in planes passing through the detail observation reference position Ps and orthogonal to each other. The three cross-sectional images Da, Db, Dc may be cross sections having the detail observation reference position Ps as a center. The center here may be a geometric center or a centroid.

In the three-dimensional image data of the dentition constituent tissue E, the plane passing through the detail observation reference position Ps is identified, and the distribution of the X-ray transmittance along the plane is obtained, whereby the three cross-sectional images Da, Db, Dc are generated. The three cross-sectional images Da, Db, Dc may be distribution of X-ray transmittance in a thick slice layer. Since the correspondence between the three-dimensional position P1 of the segmentable region and the segmentable region corresponding position P2 is identified in the arithmetic operation of the development processing, which of the coordinates of the FOV region the coordinates of the three-dimensional position P1 correspond to and/or which of the coordinates of the dentition constituent tissue region Ee the coordinates correspond to is also identified. The same applies to the coordinates of each spot in the region indicated by the dentition developed image Ep. Of course, the original coordinates arithmetic operation described above may be performed.

The correspondence between the three-dimensional position P1 of the segmentable region and the segmentable region corresponding position P2 is identified in the arithmetic operation of the development processing. Therefore, if the coordinates in the up-down and right-left directions belonging to the segmentable region can be designated in the dentition developed image Ep, it is possible to identify which one of the positions in the tangential direction and the up-down direction of the dental arch in the three-dimensional coordinate system the position designated in the dentition developed image Ep is located.

For example, in step S6, when the dentition developed image Ep in which the dentition constituent tissue E is developed is generated on the basis of the three-dimensional image data of the dentition constituent tissue E, the three-dimensional position P1 of the segmentable region is converted into the segmentable region corresponding position P2. The correspondence therebetween is stored in the storage 34 as a table. With reference to the table, which one of the tangential direction and the up-down direction of the dental arch in the three-dimensional coordinate system the position designated in the dentition developed image Ep is located can be identified. Alternatively, by inverse conversion processing of geometric conversion processing of developing the dentition constituent tissue E in the dentition developed image Ep, which one of the positions in the tangential direction and the up-down direction of the dental arch in the three-dimensional coordinate system the position designated in the dentition developed image Ep is located may be identified.

However, it may be difficult to designate the position in the buccolingual direction in the position designation with respect to the dentition developed image Ep. For example, even if the tooth of the dentition developed image Ep is designated assuming that the segmentable regions are teeth, it is unclear which position is designated in the buccolingual direction. Therefore, it is difficult to identify the detail observation reference position Ps in the three-dimensional coordinate system.

Therefore, in step S8, the detail observation reference position Ps that is a reference position for generating the detail observation image D is determined according to a predetermined rule. The rule may be a constant rule regardless of the type of the segmentable region, or may be an individual rule by type. Examples of rules are described more specifically below.

In addition, in step S8, the observation directions of the cross-sectional images Da, Db, Dc are determined. The directions of the cross-sectional images Da, Db, Dc may be any directions. For example, the three cross-sectional images Da, Db, Dc may include the cross-sectional image Dc orthogonal to the buccolingual direction, the cross-sectional image Db orthogonal to the body axis direction, and the cross-sectional image Da orthogonal to both the cross-sectional images Dc, Db. The cross-sectional image Dc orthogonal to the buccolingual direction may be a cross section along the tangential direction of the curve along the extending direction of the dentition constituent tissue E. The cross-sectional image Da orthogonal to the buccolingual direction is an example of a cross-sectional image in which the segmentable region is viewed frontally. Since what is assigned to which cross-sectional image is arbitrarily determined as the default cross-section, it is possible to appropriately change, among the cross-sectional image Da, the cross-sectional image Db, and the cross-sectional image Dc, which of the cross-sectional image orthogonal to the buccolingual direction, the cross-sectional image orthogonal to the up-down body axis direction, and the cross-sectional image orthogonal to both of these cross-sectional images is to be assigned.

A direction orthogonal to the cross-sectional images Da, Db, Dc and in which the observer observes the cross-sectional images Da, Db, Dc is defined as an observation direction. The observation directions of the cross-sectional images Da, Db, Dc may also be determined on the basis of the position information of the segmentable region. An example of determining the observation direction on the basis of the position information of the segmentable region will be described more specifically below. Note that the directions of the cross-sectional images Da, Db, Dc are directions orthogonal to the respective cross sections, and the observation direction is a direction in which the observer observes the cross-sectional images Da, Db, Dc.

The detail observation image D may be, for example, a cross-sectional image having a higher resolution than the dentition developed image Ep. The detail observation image D may be a three-dimensional image in which the line-of-sight direction can be changed.

As a result, in step S9, the detail observation image D corresponding to the position in the segmentable region designated as the display target through the user interface 24, 26 is generated on the basis of the detail observation reference position Ps described above.

In the display 22, the detail observation image D is preferably displayed on the display 22 simultaneously with the dentition developed image Ep. At this time, the position of the detail observation image D with respect to the dentition developed image Ep is arbitrary. In FIG. 11, the dentition developed image Ep and the detail observation image D are disposed laterally side by side. The dentition developed image Ep and the detail observation image D may be disposed vertically side by side. The detail observation image D may partially enter the dentition developed image Ep and be displayed in a superimposed manner.

In addition, the layout of the cross-sectional images Da, Db, Dc in the detail observation image D is arbitrary. In FIG. 11, the square display region of the detail observation image D is divided into two upward and downward and divided into two right and left. The cross-sectional image Dc orthogonal to the buccolingual direction is displayed in the lower right region, the cross-sectional image Db orthogonal to the body axis direction is displayed in the upper left region, and the cross-sectional image Da orthogonal thereto is displayed in the lower left region.

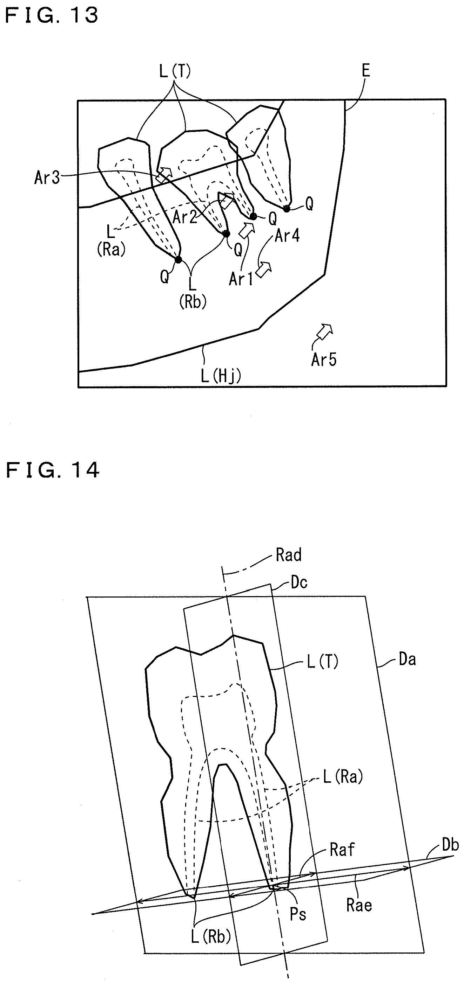

Each of the three cross-sectional images Da, Db, Dc may include a reference mark Li indicating positions of the other cross sections. The reference mark Li may be a straight line, a broken line, a short line located in the central region of the cross-sectional images Da, Db, Dc, or two marks located in the peripheral edges of the cross-sectional images Da, Db, Dc. That is, the reference mark Li may be any display that can indicate linear positions as positions of other cross sections. In FIG. 13, a vertical straight line and a horizontal straight line indicating positions of the other cross sections are illustrated as the reference mark Li in each of the cross-sectional images Da, Db, Dc.

When the display target is designated by processing from step S7 to step S9, processing of automatically displaying the detail observation image D corresponding to the designated display target is automatically executed.

Note that, even in a case where the display target is not designated, the detail observation image D corresponding to any one of the segmentable regions may be automatically displayed. Any one of the segmentable regions in which the detail observation image D is initially displayed may be a randomly selected feature region or a feature region closest to the reference position such as the upper left.

In next step S10, the processing unit 30 performs processing for display related to an adjustment operation to be described later on the detail observation image D. Specifically, step S10 is processing mainly including step S50, which is a start step of detail observation image adjustment operation-related processing to be described later, and step S53, which is a step of generating and displaying the detail observation image after adjustment.

In next step S11, the processing unit 30 determines whether another display target is designated through the user interface 24, 26. The designation of another display target may be performed similarly to that described in step S7. When it is determined that another display target is designated, the processing returns to step S8, and the subsequent processing is repeated. When it is determined that another display target is not designated, the processing proceeds to step S12, and it is determined whether the processing for diagnosis is ended. When it is input through the user interface 24, 26 or the like that the processing is ended, the processing is ended. In a case where the processing is not ended, the processing returns to step S11, and if another display target is not designated, the processing of step S12 is repeated.

Note that the designation of the display target is designation of the segmentable region corresponding position. The designation of the segmentable region corresponding position can be said to be designation of any one of the plurality of segmentable regions. The designation of the segmentable region corresponding position may be considered to include designation of a coordinate position in the designated segmentable region in the two-dimensional coordinate system of the dentition developed image Ep.

### <Example of determination processing of detail observation reference position and cross-sectional direction>

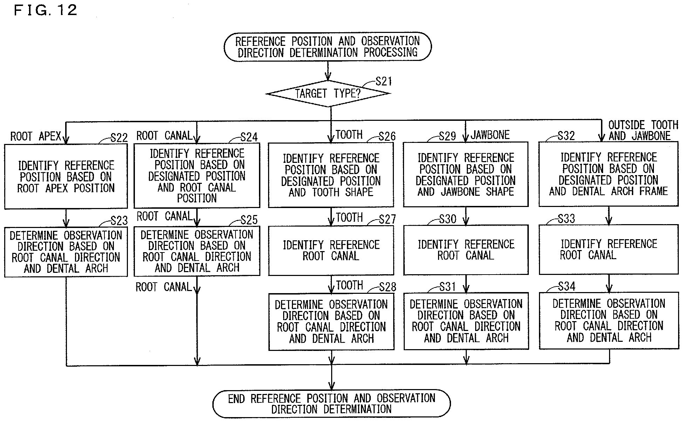

With reference to the flowchart illustrated in FIG. 12, the processing of above step S8 will be described more specifically. Here, an example in which the detail observation reference position Ps and the cross-sectional direction are individually determined by type of the segmentable region will be described.

As a premise, as illustrated in FIG. 13, it is assumed that the three-dimensional positions P1 of the teeth T, the jawbone Hj, root canals Ra, and root apexes Rb are identified as the segmentable regions L, and the segmentable region corresponding positions P2 of the teeth T, the jawbone Hj, the root canals Ra, and the root apexes Rb are also identified in the dentition developed image Ep.

Since the root apex Rb exists at a limited location, the three-dimensional position P1 is identified by representative point coordinates. In addition, since the root canal Ra is an elongated portion, the three-dimensional position P1 may be identified by a line. For the tooth T and the jawbone Hj, it is assumed that the three-dimensional position P1 is identified by the data of the surface or the data indicating the solid in which the interior is filled.

When the display target is designated using the user interface 24, 26, a type of the designation target is determined in step S21. The type is differentiated as the tooth T, the jawbone Hj, the root canal Ra, the root apex Rb, and others which are identified as the segmentable region. For example, it is determined whether the position designated by a key, a mouse, or the like with respect to the dentition developed image Ep belongs to any one of the tooth T, the jawbone Hj, the root canal Ra, the root apex Rb, and other regions, whereby it is determined whether the designated position belongs to any one of the above types. A rule for determining the detail observation reference position Ps is individually set in advance by type described above, and is incorporated in the processing of steps S22, S24, S26, and S29.

When it is determined in step S21 that the target is the root apex Rb, the processing proceeds to step S22 (See an arrow Ar1 in FIG. 13).

In step S22, the reference position is identified on the basis of the three-dimensional position P1 of the root apex Rb. That is, when any one of the root apexes Rb is identified in the dentition developed image Ep, the three-dimensional position P1 corresponding to the root apex Rb is identified (See FIG. 14). Since the root apex Rb exists at a limited location, the three-dimensional direction is identified by representative point coordinates. Therefore, the three-dimensional position P1 of the root apex Rb can be set as the detail observation reference position Ps.

Step S22 is an example of processing performed according to the rule of determining the detail observation reference position Ps on the basis of the position information of at least a part of the root apex Rb that is the segmentable region.

In next step S23, the observation direction is determined on the basis of the direction of the root canal Ra and the dental arch. For example, since the root apex Rb is located at the tip of the root canal Ra, the root canal Ra from which the designated apex Rb extends can be identified on the basis of the three-dimensional position of the root apex Rb and the three-dimensional position of the root canal Ra. Then, a root canal direction Rad in which the root canal Ra extends can be identified on the basis of the data indicating the line that is the three-dimensional position of the root apex Rb.

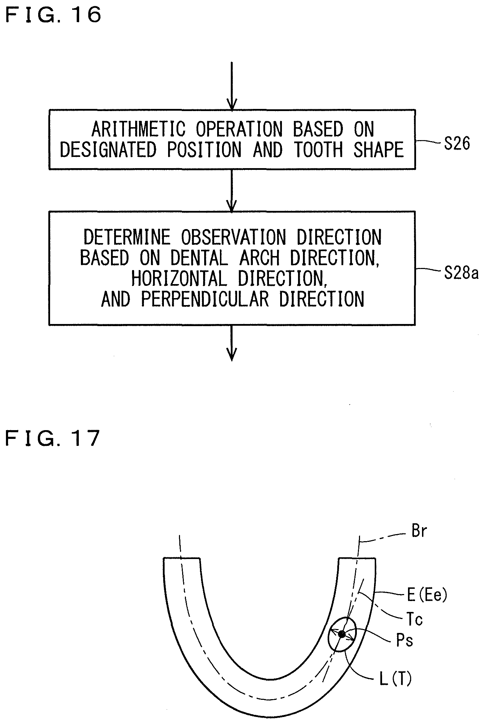

In addition, at each coordinate position in the up-down direction, a curve having an arch shape or a horseshoe shape passing through the center in the buccolingual direction of the dentition constituent tissue E, that is, a dental arch curve, is calculated (See FIG. 17). A tangential direction Rae at the position closest to the detail observation reference position Ps in the dental arch curve is calculated. The tangential direction Rae may directionally coincide with a tangential direction Tc in FIG. 17 to be described later. A direction orthogonal to both the root canal direction Rad and the tangential direction Rae is defined as a multi-orthogonal direction Raf. Since the root canal direction Rad is generally a direction substantially parallel to the body axis, the multi-orthogonal direction Raf coincides or substantially coincides with the buccolingual direction in many cases.

For example, the observation directions of the three cross-sectional images Da, Db, Dc can be the multi-orthogonal direction Raf, the root canal direction Rad, and the tangential direction Rae.

The multi-orthogonal direction Raf is preferably a direction in which the target portion is viewed frontally from the outside to the inside. The root canal direction Rad may be a direction toward the jawbone side or a direction away from the jawbone. The tangential direction Rae may be either rightward or leftward.

In each of the following examples, the observation direction of each cross section is preferably a direction in which the target portion is viewed frontally from the outside to the inside in the buccolingual direction, and may be any direction in the up-down and right-left directions.

In this manner, by determining the observation direction on the basis of the direction of the root canal Ra, the observation direction of the detail observation image D is determined on the basis of the position information of at least a part of the root canal Ra which is the segmentable region identified in the dentition constituent tissue E. Here, the segmentable region used to determine the observation direction does not need to be the segmentable region designated as the display target, and may be an adjacent region or a neighboring region of the segmentable region designated as the display target. When the observation direction of the root canal Ra, the tooth T, the jawbone Hj, and the like described below is determined, the observation direction is determined on the basis of the position information of the display target or the segmentable region near the display target.

As a result, the detail observation reference position Ps and the observation direction for the detail observation image D are determined. In step S9, the detail observation image D is generated on the basis of the detail observation reference position Ps and the observation direction, and is displayed on the display 22.

In this case, since the cross section of the root canal Ra appears in the cross-sectional images Da, Dc along the root canal direction, it is easy to observe the living body from the root apex Rb to the root canal Ra.

When it is determined in step S21 that the target is the root canal Ra, the processing proceeds to step S24 (See an arrow Ar2 in FIG. 13).

In step S24, the reference position is identified on the basis of the designated position with respect to the root canal Ra in the dentition developed image Ep and the three-dimensional position P1 of the root canal Ra.

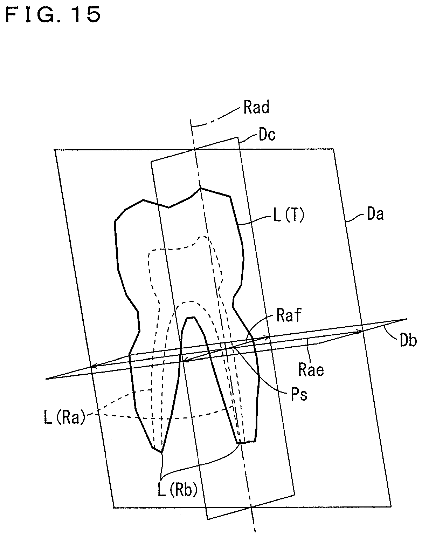

For example, it is assumed that any one of the root canals Ra is identified in the dentition developed image Ep, and a position of one portion in the extending direction of the root canal Ra is designated. The position of the root canal Ra in the extending direction can be grasped as a position in the up-down direction. Therefore, as illustrated in FIG. 15, the three-dimensional position of the detail observation reference position Ps can be identified on the basis of the correspondence between the three-dimensional position P1 and the segmentable region corresponding position P2 with respect to the designated root canal Ra and the upper and lower designated positions in the root canal Ra.

That is, when any root canal Ra is designated in the dentition developed image Ep, the position in the buccolingual direction can be determined on the basis of the three-dimensional position P1 of the root canal Ra.

Step S24 is an example of processing performed according to the rule of determining the detail observation reference position Ps on the basis of the position information of at least a part of the root canal Ra that is the segmentable region.

In next step S25, the observation direction is determined on the basis of the direction of the designated root canal Ra and the dental arch. The determination of the observation direction based on the direction of the designated root canal Ra and the dental arch can be performed similarly to step S23.

When it is determined in step S21 that the target is the tooth T, the processing proceeds to step S26 (See an arrow Ar3 in FIG. 13).

In step S26, the reference position is identified on the basis of the designated position in the region of the tooth T in the dentition developed image Ep and the shape of the tooth T.

For example, when the position in the region of the tooth T is designated in the dentition developed image Ep, the designated position is converted into the position in the three-dimensional coordinate system by a table that defines the correspondence between the three-dimensional position P1 of the segmentable region and the segmentable region corresponding position P2 or inverse conversion processing of geometric conversion processing of developing the dentition constituent tissue E in the dentition developed image Ep. However, in the position designation with respect to the tooth T in the dentition developed image Ep, since it is difficult to designate the position in the buccolingual direction, the converted position in the three-dimensional coordinate system also lacks information on the position in the buccolingual direction. That is, based on the position designation in the region of the tooth T in the dentition developed image Ep, the positions in the tangential direction and the up-down direction of the dental arch curve of the tooth T in the three-dimensional coordinate system can be identified, but the position in the buccolingual direction cannot be identified.

Therefore, for the buccolingual direction, the position is identified on the basis of the shape of the tooth T. For example, a line passing through a designated position in the three-dimensional coordinate system penetrates the tooth T in the buccolingual direction. In the line, the center of the boundary of the tooth T is set as the reference position in the buccolingual direction at the detail observation reference position Ps.

As a result, the three dimensions of the detail observation reference position Ps can be determined.

The reference position in the buccolingual direction at the detail observation reference position Ps is not limited to the above position. For example, the reference position in the buccolingual direction at the detail observation reference position Ps may be the position of the root canal Ra located closest thereto or the position of the dental arch curve.

Step S26 is an example of processing performed according to the rule of determining the detail observation reference position Ps on the basis of the position information of at least a part of the tooth T that is the segmentable region.

In next step S27, a reference root canal is identified in order to determine the observation direction. The reference root canal may be set to the root canal Ra of the tooth T to which the designated position belongs, or the root canal Ra closest to the designated position.

In next step S28, with reference to the reference root canal, the observation direction is determined on the basis of the direction of the root canal Ra and the dental arch. The determination of the observation direction based on the direction of the designated root canal Ra and the dental arch can be performed similarly to step S23.

Note that the observation direction may be determined not based on the direction of the root canal Ra. For example, as illustrated in FIG. 16, after step S26, step S28a may be executed instead of steps S27 and S28.

In step S28a, the observation direction is determined on the basis of the dental arch curve, the horizontal direction, and the vertical direction. As illustrated in FIG. 17, the tangential direction Tc at the position closest to the detail observation reference position Ps in a dental arch curve Br is calculated. In addition, a horizontal direction Ta and a vertical direction Tb orthogonal to the tangential direction Tc are calculated.

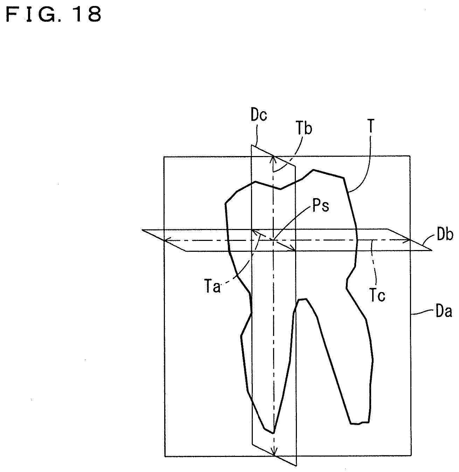

Then, as illustrated in FIG. 18, the horizontal direction Ta, the vertical direction Tb, and the tangential direction Tc are set as observation directions of the cross-sectional images Da, Db, and Dc, respectively. The vertical direction is a gravity direction, and the horizontal direction is a direction orthogonal to the gravity direction.

The processing of determining the observation directions of the cross-sectional images Da, Db, Dc not based on the direction of the root canal Ra may be applied as processing for determining the observation directions for other root apexes Rb, root canals Ra, jawbone Hj, jawbone Hj outer region, and the like.

When it is determined in step S21 that the target is the jawbone Hj, the processing proceeds to step S29 (See an arrow Ar4 in FIG. 13).

In step S29, the reference position is identified on the basis of the designated position in the region of the jawbone Hj in the dentition developed image Ep and the shape of the jawbone Hj.

For example, when the position in the region of the jawbone Hj is designated in the dentition developed image Ep, the designated position is converted into the position in the three-dimensional coordinate system by a table that defines the correspondence between the three-dimensional position P1 of the segmentable region and the segmentable region corresponding position P2 or inverse conversion processing of geometric conversion processing of developing the dentition constituent tissue E in the dentition developed image Ep. However, similarly to the case of the position designation with respect to the tooth T, it is difficult to designate the position in the buccolingual direction in the position designation with respect to the jawbone Hj in the dentition developed image Ep. Therefore, for the buccolingual direction, the position is identified on the basis of the shape of the jawbone Hj. Similarly to the case of the tooth T, the position in the buccolingual direction may be identified by setting the center of the jawbone Hj in the buccolingual direction as the reference position in the buccolingual direction at the detail observation reference position Ps.

As a result, the three-dimensional coordinates of the detail observation reference position Ps can be determined.

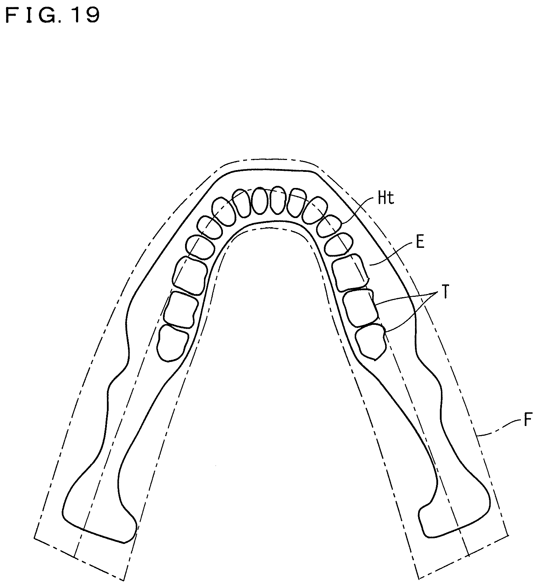

The reference position in the buccolingual direction at the detail observation reference position Ps is not limited to the above position. For example, the reference position in the buccolingual direction at the detail observation reference position Ps may be the position of the root canal Ra located closest thereto or the position of the dental arch curve. In addition, for example, as illustrated in FIG. 19, the horseshoe-shaped frame F that can include the jawbone Hj can be set for the three-dimensional image data including the dentition constituent tissue E. The horseshoe-shaped frame F is a frame preset as a shape and a size that can include the general dentition constituent tissue E. For example, the horseshoe-shaped frame F is set with respect to the three-dimensional image data such that the dentition constituent tissue E is accommodated in the horseshoe-shaped frame F by an image alignment algorithm or the like. Alternatively, the head holder 9 sets the dentition constituent tissue E of the standard physique to be disposed at a predetermined position in the three-dimensional image data, and the horseshoe-shaped frame F is set at a position that can include the dentition constituent tissue E in the three-dimensional image data. A line FL passing through the center of the horseshoe-shaped frame F in the thickness direction may be set in advance, and the position on the line FL may be set as the position in the buccolingual direction. Of course, the line FL may be set closer to the buccal side or closer to the lingual side according to the need or desire of the user.

Step S29 is an example of processing performed according to the rule of determining the detail observation reference position Ps on the basis of the position information of at least a part of the jawbone Hj that is the segmentable region.

In next step S30, the reference root canal is identified in order to determine the observation direction. The reference root canal may be set to the root canal Ra closest to the designated position.

In next step S31, with reference to the reference root canal, the observation direction is determined on the basis of the direction of the root canal Ra and the dental arch. The determination of the observation direction based on the direction of the designated root canal Ra and the dental arch can be performed similarly to step S23.

The observation direction may be determined on the basis of the tangential direction of the curved line in which the jawbone Hj extends.

When it is determined in step S21 that the target is not the tooth T and the jawbone Hj, the processing proceeds to step S32 (See an arrow Ar5 in FIG. 13).

In step S32, the reference position is identified on the basis of the designated position in the region outside the jawbone Hj in the dentition developed image Ep and the horseshoe-shaped frame F.

For example, when the position in the region exterior to the jawbone Hj is designated in the dentition developed image Ep, the designated position is converted into the position in the three-dimensional coordinate system by inverse conversion processing of geometric conversion processing of developing the dentition constituent tissue E in the dentition developed image Ep. For the buccolingual direction, the position may be determined using the horseshoe-shaped frame F. In addition, the center in the buccolingual direction of the jawbone Hj closest to the designated position may be set as the position in the buccolingual direction.

As a result, the three-dimensional coordinates of the detail observation reference position Ps can be determined.

In next step S33, the reference root canal is identified in order to determine the observation direction. The reference root canal may be set to the root canal Ra closest to the designated position.

In next step S34, with reference to the reference root canal, the observation direction is determined on the basis of the direction of the root canal Ra and the dental arch. The determination of the observation direction based on the direction of the designated root canal Ra and the dental arch can be performed similarly to step S23.

The observation direction may be determined on the basis of the tangential direction of the curved line of the jawbone Hj closest to the designated position or the tangential direction of the dental arch curve.

As described above, in the determination of the observation direction, a root canal that satisfies a positional relationship condition with a predetermined designated position, such as being closest to the designated position, can be used as the reference root canal, and the processing of determining the observation direction may include processing of determining the observation direction on the basis of the root canal direction in which the reference root canal extends. When a root apex is designated, a root canal connected to the root apex may be the reference root canal, and when a root canal is designated, the designated root canal itself may be the reference root canal.

That is, in the processing of generating the detail observation image of the segmentable region from the three-dimensional image data on the basis of the correspondence between the segmentable region corresponding position and the three-dimensional position by the designation of the segmentable region corresponding position in the dentition developed image, and determining the detail observation reference position that is the reference position for generating the detail observation image according to a predetermined rule, the processing of determining the observation direction of the detail observation image on the basis of the position information of at least a part of the segmentable region identified in the dentition constituent tissue is performed, and the processing of determining the observation direction may be processing of determining the observation direction on the basis of the root canal direction of the reference root canal by using, as the reference root canal, a root canal that satisfies the positional relationship condition with the designated position of the designation operation via the user interface.

In this manner, the detail observation reference position Ps and the observation direction are individually determined by type of the target. Thereafter, in step S9, the detail observation image D is generated on the basis of the determined detail observation reference position Ps and observation direction, and is displayed on the display 22.

With respect to the tooth T and the jawbone Hj, by setting the center in the buccolingual direction as the detail observation reference position Ps, the detail observation reference position Ps is set at a position within the anatomical differentiation region.

### <Example of display processing on detail observation image>

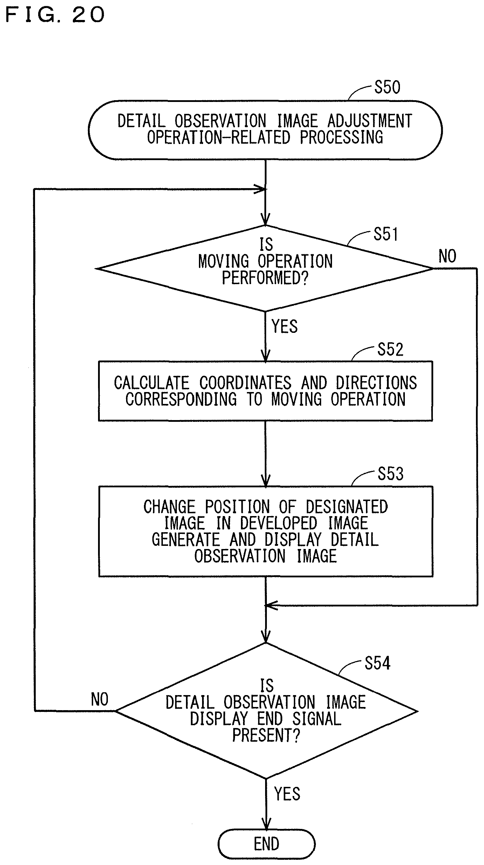

An example of display processing on the detail observation image D will be described. FIG. 20 is a flowchart illustrating a specific example of processing of step S10 in FIG. 4.

When the detail observation image D is displayed on the display 22, the cross-sectional positions of the cross-sectional images Da, Db, Dc of the detail observation image D are changed in response to the moving operation received through the user interface 24, 26.

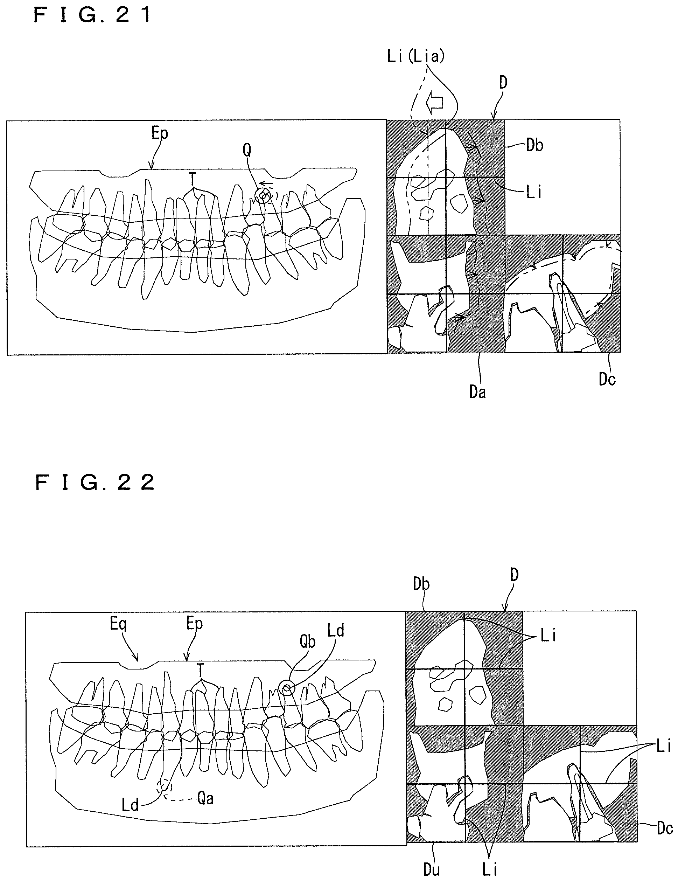

That is, after the detail observation image D is displayed, the processing proceeds to step S50, the detail observation image adjustment operation-related processing is started, and step S51 is processed. In step S51, in the processing unit 30, whether the moving operation is performed is determined. The moving operation is received, for example, by an operation on the reference mark Li through the user interface 24, 26. For example, as illustrated in FIG. 21, by moving any reference mark Li (referred to as reference mark Lia in FIG. 21) with a pointer device such as a mouse, the cross-sectional position indicated by the reference mark Lia is moved. As a result, the moving operation is received. The moving operation may be performed by a switch device such as a keyboard.

The moving operation in step S51 may be an operation of changing the inclination of the reference mark Li. For example, in a case where the central region of the reference mark Li is subjected to a drag operation by a pointer device such as a mouse, the position of the reference mark Li may be changed, and, in a case where the end region of the reference mark Li is subjected to the drag operation by a pointer device such as a mouse, the inclination of the reference mark Li may be changed. The rotation operation of the three-dimensional image may be performed by applying a point operation to a portion deviating from the reference mark Li of the cross-sectional image Da, the cross-sectional image Db, and the cross-sectional image Dc. The inclination of the reference mark Li is changed according to the direction and amount of the drag operation, and a moving operation of inclining the cross-sectional position is received.

When it is determined in step S51 that the moving operation is not performed, the processing proceeds to step S54, and when it is determined that the moving operation is performed, the processing proceeds to step S52.

In step S52, coordinates and a direction corresponding to the moving operation are calculated. The coordinates corresponding to the moving operation are coordinates obtained by shifting the coordinates of the three-dimensional position P1 before the movement by the movement amount corresponding to the moving operation. By converting the coordinates after the moving operation by processing similar to the processing of converting the three-dimensional position P1 into the segmentable region corresponding position P2, the coordinates of the segmentable region after the moving operation in the dentition developed image Ep are calculated. In addition, the direction corresponding to the moving operation is a direction obtained by inclining a direction orthogonal to any of the cross-sectional images Da, Db, Dc at the three-dimensional position P1 before the movement by an inclination corresponding to the moving operation.

The process of step S53 may be provided after step S52. In step S53, the processing unit 30 changes the position of the image indicating the position designated in the dentition developed image Ep to the position after the moving operation. Furthermore, the cross-sectional images Da, Db, Dc are generated and displayed using the coordinates and the direction after the moving operation as references. After the moving operation, the cross-sectional images Da, Db, Dc are generated so as to be disposed around the coordinates after the moving operation as a center. Therefore, in the cross-sectional images Da, Db, Dc, the image before the operation is displayed with being shifted to the opposite side to the moving operation direction, or the image in the depth or front direction of the image before the operation is displayed (See the outline indicated by the two-dot chain line in FIG. 13). The processing of step S53 may be configured to be optionally executable so that the user can select whether to proceed to step S53 through the user interface 24, 26. In this case, it may be possible to skip step S53. In addition, the process itself of step S53 may be omitted.

When step S53 ends, the processing proceeds to step S54 (In a case where the processing of step S53 is omitted, the processing proceeds to step S54 when step S52 ends).

In step S54, the presence or absence of a signal to end the display of the detail observation image D is determined. The signal to end the display is transmitted, for example, when the user gives an instruction to end the display through the user interface 24, 26. In a case where a signal to end the display is present, the display of the detail observation image D ends. In a case where the signal to end the display is absent, the processing returns to the processing of step S51.

### <Effects and the like>

According to the image processing apparatus 20, the image processing method, and the program 34a configured as described above, the detail observation reference position Ps of the segmentable region corresponding position P2 is generated on the basis of the correspondence between the segmentable region corresponding position P2 and the three-dimensional position P1 by the designation of the segmentable region corresponding position P2 in the dentition developed image Ep, and is displayed on the display 22. At this time, the detail observation reference position Ps that is a reference position for generating the detail observation image D is determined according to a predetermined rule. Accordingly, the position of the detail observation image D can be appropriately set in the dentition developed image Ep. For example, even if it is difficult to designate the detail observation reference position Ps in the buccolingual direction in the dentition developed image Ep, the detail observation reference position Ps subjected to position designation can be appropriately set also in the buccolingual direction.

In addition, since the above rule is a rule for determining the detail observation reference position Ps on the basis of the position information of at least a part of the segmentable region, the detail observation reference position Ps is determined according to the position of the segmentable region.

In addition, by determining the observation direction of the detail observation image D on the basis of the position information of at least a part of the segmentable region identified in the dentition constituent tissue E, it is possible to generate the detail observation image D that is easily observed in detail. For example, the detail observation image D along the direction of the root canal Ra is generated on the basis of the position information of the root canal Ra as the segmentable region, so that the root canal Ra can be easily observed in detail.

In addition, in the dentition developed image Ep, it is difficult to set the position in the buccolingual direction. Therefore, the detail observation reference position Ps in the buccolingual direction is determined according to a predetermined rule. Accordingly, the position of the detail observation image D can be appropriately set in the buccolingual direction.

In addition, the type of the segmentable region is identified, and the rule is individually set by type. Accordingly, the detail observation reference position Ps suitable by type can be individually set. For example, if the segmentable region is the root apex Rb, the detail observation reference position Ps can be set at the position of the root apex Rb itself, and if the segmentable region is the root canal Ra, the detail observation reference position Ps can be set at the position along the root canal Ra. If the segmentable region is the tooth T, the detail observation reference position Ps can be set at the central position of the tooth T in the buccolingual direction.

In addition, for example, if the segmentable region is a lesion region, by setting the center of the lesion region as the detail observation reference position Ps, it is easy to observe a portion extending from the center of the lesion region. The center may be, for example, a geometric center or a centroid.

In addition, by setting the anatomical differentiation regions such as the teeth T, the root canals Ra, the root apexes Rb, and the jawbones Hj as the segmentable regions, the position of the detail observation image D with respect to the anatomical differentiation region can be appropriately set.

Furthermore, by setting the central position in the buccolingual direction with respect to the teeth T or the jawbone Hj as the detail observation reference position Ps, the internal structure of the anatomical differentiation region can be observed using the detail observation image D.

In addition, if the detail observation image D includes three cross-sectional images Da, Db, Dc orthogonal to each other, it is easy to observe the segmentable region in detail.

In addition, the rule may set the observation directions of the cross-sectional images Da, Db, Dc by type of the segmentable region. For example, as described above, the observation direction may be determined with reference to the root canal Ra for the root apex Rb and the root canal Ra, and the observation direction may be determined with reference to the horizontal direction and the perpendicular direction without reference to the root canal Ra for the tooth T and the jawbone Hj (see FIG. 18).

As a result, the direction of the cross-sectional image can be individually set by type of the segmentable region.

In addition, by identifying a plurality of segmentable regions, displaying, on the display 22, the dentition developed image Ep in which the plurality of segmentable regions are displayed at the segmentable region corresponding positions, receiving designation of some of the plurality of segmentable regions through the user interface 24, 26, and displaying, on the display 22, the detail observation images D respectively corresponding to the some segmentable regions received, the detail observation images respectively corresponding to the some segmentable regions designated from the plurality of segmentable regions can be observed in detail.

In addition, by changing the detail observation reference position Ps in response to the reference position change operation through the user interface 24, 26, it is easy to observe the segmentable region at various positions.

In the present preferred embodiment, the detail observation reference position Ps can be changed by a continuous position designation operation on the dentition developed image Ep or by an operation on the detail observation image D.

### {Modifications}

Various modifications will be described on the premise of the above preferred embodiment.

In the above preferred embodiment, an example in which the segmentable region is the anatomical differentiation region has been mainly described. As illustrated in FIG. 22, the segmentable region may be a biologically characteristic region, for example, a lesion region Ld.

The identification of the lesion region Ld may be performed by applying a learned machine learning model similar to that in the identification of the dentition constituent tissue. For example, as training data, a large number of pieces of data in which regions of lesions are mapped to the three-dimensional volume data are prepared. A machine learning model learned to segment the regions of lesions in the three-dimensional volume data is prepared using the training data. By applying the learned machine learning model to the three-dimensional volume data, lesion regions in the three-dimensional coordinate system are identified.

The identification of the lesion region in the three-dimensional volume data may be performed by image extraction processing, for example, pattern matching processing.

The detection of the lesion region may not be performed on the basis of the three-dimensional volume data. For example, the lesion region may be identified by applying a machine learning model, pattern matching, or the like on the basis of slice data sliced in any direction of three-dimensional volume data.

There is a possibility that the lesion region Ld is not displayed as an image that can be clearly differentiated from others in the dentition developed image Ep. Therefore, for the lesion region Ld, the annotation images Qa, Qb may be displayed at the segmentable region corresponding positions P2 in the dentition developed image Ep.

The annotation images Qa, Qb are images indicating the segmentable regions. The annotation images Qa, Qb may be images exhibiting a color, a shape, or a change that enables differentiation from the dentition developed image Ep spreading in the background. For example, the annotation images Qa, Qb may be images exhibiting a hue different from that of the dentition developed image Ep. More specifically, the annotation images Qa, Qb may be images exhibiting a warm color that is easily noticeable, for example, red, orange, or yellow. The annotation images Qa, Qb may be images with a display change that enables differentiation from the dentition developed image Ep, for example, blinking, a color change, or a shape change. The annotation images Qa, Qb may have a shape that enables differentiation from the dentition developed image Ep, for example, a circle, a regular polygon, a radial shape, a cross shape, an exclamation mark shape, or the like.

The annotation images Qa, Qb may have a shape indicating the outline of the lesion region Ld. In this case, it is possible to know the approximate shape and size of the lesion region Ld by viewing the annotation images Qa, Qb.

In a case where a plurality of segmentable regions are detected, the annotation images Qa, Qb are displayed in a recognizable manner correspondingly to the plurality of segmentable regions, respectively.

Note that, the case where the plurality of annotation images Qa, Qb are displayed in a recognizable manner includes not only the case where the plurality of annotation images Qa, Qb are simultaneously displayed, but also the case where the plurality of annotation images Qa, Qb are sequentially displayed to the extent that the plurality of annotation images Qa, Qb can be recognized. For example, the plurality of annotation images Qa, Qb may be sequentially displayed within 5 seconds, 3 seconds, or 1 second.

In a case where a plurality of annotation images Qa, Qb are present, one of the plurality of annotation images Qa, Qb is designated, whereby the detail observation image D corresponding to the designated image Qa, Qb is displayed. The designated annotation image Qb may be displayed so as to be differentiated from the other annotation image Qa. In FIG. 22, the designated annotation image Qb is displayed with a double circle, and the other annotation image Qa is displayed with a single circle.

An image in which the annotation images Qa, Qb are superimposed on the dentition developed image Ep is a segmentable region arrangement developed image Eq.

For the segmentable region such as the lesion region Ld, the center of the segmentable region may be set as the detail observation reference position Ps. The center may be the center of the region identified as the segmentable region, for example, a geometric center or a centroid.

According to this modification, the position of the detail observation image D with respect to the segmentable region can be appropriately set.

Furthermore, by displaying the annotation images Qa, Qb so as to indicate the segmentable regions, it is easy to recognize the segmentable regions such as the lesion regions Ld using the annotation images Qa, Qb as a clue.

Furthermore, by setting the center of the segmentable region such as the lesion region Ld as the detail observation reference position Ps, observation can be performed using the detail observation image D with the center of the segmentable region as a reference.

In the dentition developed image Ep, the segmentable regions may be displayed in different display modes by type. For example, the tooth T and the jawbone Hj may be displayed in different colors.

In the above preferred embodiment, an example has been described in which each position of the dentition constituent tissue E is identified on the basis of the three-dimensional volume data and developed in the dentition developed image Ep on the basis of the identified dentition constituent tissue E.

However, respective positions of the dentition constituent tissue E may not be individually identified on the basis of the three-dimensional volume data, and may be developed in the dentition developed image Ep on the basis of the regions of the standard dentition constituent tissue E.

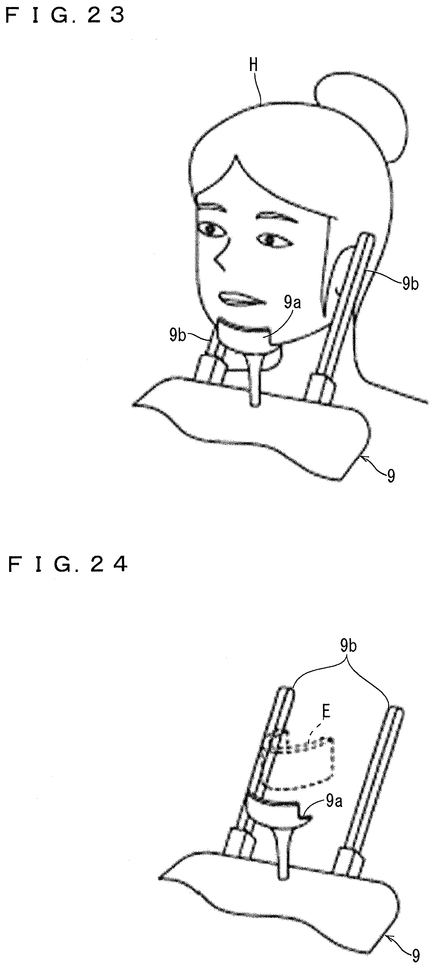

For example, as illustrated in FIG. 23, the head H is positioned at a fixed position by the head holder 9 when CT imaging is performed. As a result, as illustrated in FIG. 24, the dentition constituent tissue E is also positioned at a fixed position with respect to the head holder 9.

The positional relationship between the head holder 9 and the X-ray generator 11 and X-ray detector 12 can be a known positional relationship. In addition, the region in which the dentition constituent tissue E spreads in the head H may be considered to be constant to some extent. Therefore, in the three-dimensional volume data based on the CT imaging data, the region of the standard dentition constituent tissue E may be treated as the region of the actual dentition constituent tissue E.

In addition, on the premise of the standard dentition constituent tissue E, it is possible to determine in advance general-purpose conversion processing of developing the standard dentition constituent tissue E in the dentition developed image Ep. The region where the dentition constituent tissue E is assumed to be present in the three-dimensional volume data can be developed in the dentition developed image Ep by the general-purpose conversion processing. In addition, by performing general-purpose conversion processing on the identified segmentable region in the three-dimensional volume data, the corresponding position of the segmentable region in the dentition developed image Ep can be calculated.

In the above preferred embodiment, the case where the image processing apparatus 20 is used with the CT imaging apparatus 10 as a set has been described.

The image processing apparatus 20 may be configured as an apparatus separate from the CT imaging apparatus 10.

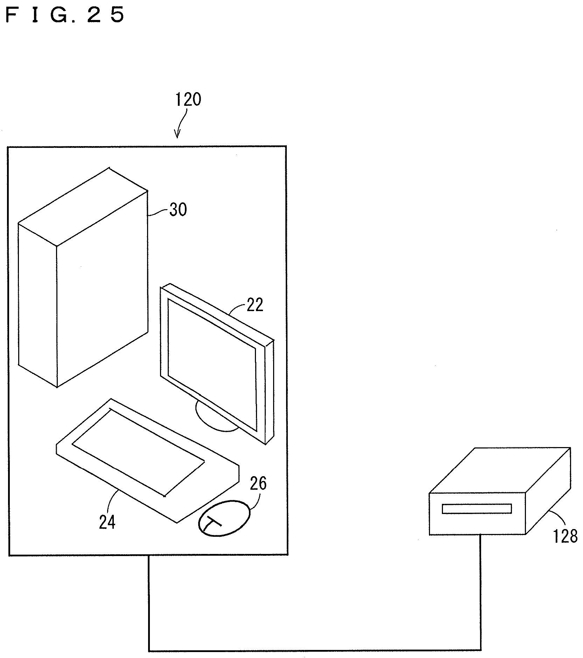

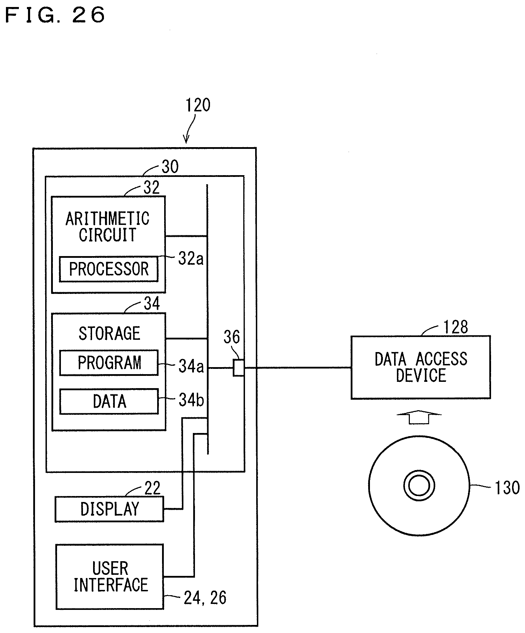

In this case, as in an image processing apparatus 120 illustrated in FIG. 25 and FIG. 26, the image processing apparatus 120 may include a data access device 130 connected through the connection port 36. The data access device 130 is, for example, a device capable of reading data recorded on a recording medium 128. The recording medium 128 may be an optical recording medium such as an optical disk, or may be a flash memory such as a USB memory or an SD card. The data access device 130 may be a reading device for an optical disk, or a card reader.

Image data obtained by CT imaging is recorded in the recording medium 128. The image data may be an X-ray projection image data group, three-dimensional volume data, or a data group of a plurality of tomographic images.

The image processing apparatus 120 can execute processing similar to that of the above preferred embodiment by reading image data obtained by CT imaging via the data access device 130.

The image data obtained by the CT imaging is stored in the server apparatus, and the image processing apparatus 120 may access the server apparatus through the communication network to obtain the image data obtained by the CT imaging. In this case, the server apparatus may be located in a facility where the image processing apparatus 120 exists or may be located outside the facility. The server apparatus may be a cloud server. The image processing apparatus 120 can access the server apparatus through a dedicated line or a public line network to acquire image data obtained by CT imaging.

### {Other modifications}

In the above preferred embodiment and various modifications, other information may be displayed around the dentition developed image Ep or the detail observation image D. For example, a disease name may be described. For example, in a case where a plurality of lesion regions Ld are detected, the respective disease names of the plurality of lesion regions may be displayed. The disease names may be displayed together with the cross-sectional images.

Note that the configurations described in the above preferred embodiment and modifications can be appropriately combined as long as they do not contradict each other.

The present disclosure discloses the following aspects.

A first aspect is an image processing apparatus that processes image data obtained by computed tomography (CT) imaging of dentition constituent tissue to generate an image for diagnosis, the image processing apparatus including: a storage that stores the image data; a processor; and a display that displays an observation image on the basis of output of the processor, wherein the processor generates three-dimensional image data of the dentition constituent tissue on the basis of the image data, identifies a segmentable region in the dentition constituent tissue on the basis of the image data, identifies a three-dimensional position at which the segmentable region is present in a coordinate system of the three-dimensional image, generates a dentition developed image in which the dentition constituent tissue is developed on the basis of the three-dimensional image data, identifies a segmentable region corresponding position corresponding to the three-dimensional position in the dentition developed image, displays, on the display, the dentition developed image in which the segmentable region is displayed at the segmentable region corresponding position, generates a detail observation image of the segmentable region from the three-dimensional image data on the basis of a correspondence between the segmentable region corresponding position and the three-dimensional position by designation of the segmentable region corresponding position in the dentition developed image, determines a detail observation reference position that is a reference position for generating the detail observation image according to a predetermined rule, and displays the detail observation image on the display.

According to this image processing apparatus, the detail observation reference position of the segmentable region corresponding position is generated on the basis of the correspondence between the segmentable region corresponding position and the three-dimensional position by the designation of the segmentable region corresponding position in the dentition developed image, and is displayed on the display. At this time, the detail observation reference position that is a reference position for generating the detail observation image is determined according to a predetermined rule. Therefore, the position of the detail observation image can be appropriately set in the dentition developed image.

The processor may set dentition constituent tissue region that is a region in which the dentition constituent tissue spreads and a dentition developed region in which the dentition constituent tissue region is developed, and the development of the dentition constituent tissue may be performed by development processing of developing image data of the dentition constituent tissue in the dentition constituent tissue region so as to conform to a shape of the dentition developed region. As a result, appropriate image data for visual recognition of the segmentable region can be processed.

The development processing may be performed such that the buccolingual direction in the pre-development curved state, which is the state of the dentition constituent tissue region before development, corresponds to the frontally-viewing direction in the post-development at-a-glance state, which is the state of the dentition developed region after development. This makes it easy to understand the position of the segmentable region.

Setting may be made such that the dentition constituent tissue region has a thickness in the buccolingual direction and the dentition developed region has a thickness in the frontally-viewing direction corresponding to the buccolingual direction. As a result, it is possible to recognize the segmentable region within an appropriate range.

In the development described above, an original coordinates arithmetic operation for associating the segmentable region with the three-dimensional position of the segmentable region corresponding position may be performed. In the original coordinates arithmetic operation, arithmetic operation of identifying which position in the buccolingual direction the three-dimensional position of the segmentable region is located and identifying which position in the frontally-viewing direction corresponding to the buccolingual direction the segmentable region corresponding position is located may be performed. As a result, it is possible to calculate with accuracy the three-dimensional position of the segmentable region and the segmentable region corresponding position.

A second aspect is the image processing apparatus according to the first aspect, wherein the rule is a rule for determining the detail observation reference position on the basis of position information of at least a part of the segmentable region.

As a result, the detail observation reference position can be determined on the basis of the position information of at least a part of the segmentable region.

A third aspect is the image processing apparatus according to the first or second aspect, wherein the processor determines an observation direction of the detail observation image on the basis of position information of at least a part of the segmentable region identified in the dentition constituent tissue.

In this case, since the observation direction of the detail observation image can be determined on the basis of the position information of at least a part of the segmentable region or the adjacent region of the segmentable region, it is easy to perform detailed observation.

A fourth aspect is the image processing apparatus according to any one of the first to third aspects, wherein the detail observation reference position includes a position in a buccolingual direction.

In the dentition developed image, it is difficult to set the position in the buccolingual direction. Therefore, the detail observation reference position in the buccolingual direction is determined according to a predetermined rule. Accordingly, the position of the detail observation image can be appropriately set in the buccolingual direction.

A fifth aspect is the image processing apparatus according to any one of the first to fourth aspects, wherein the processor identifies a type of the segmentable region, and the rule is individually preset by type.

Accordingly, the detail observation reference position suitable for each type can be individually set.

A sixth aspect is the image processing apparatus according to any one of the first to fifth aspects, wherein the processor detects a biological feature region in the dentition constituent tissue on the basis of the image data, and sets the biological feature region as the segmentable region.

Accordingly, the position of the detail observation image with respect to the biological feature region can be appropriately set.

A seventh aspect is the image processing apparatus according to the sixth aspect, wherein the processor displays, on the display, a biological feature region arrangement developed image in which an annotation image indicating the biological feature region is superimposed on the dentition developed image such that the annotation image is located at the segmentable region corresponding position.

As a result, it is easy to recognize the biological feature region using the annotation image as a clue.

An eighth aspect is the image processing apparatus according to the sixth or seventh aspect wherein the processor sets a center of the biological feature region as the detail observation reference position.

As a result, observation can be performed using the detail observation image with the center of the biological feature region as a reference.

A ninth aspect is the image processing apparatus according to any one of the first to eighth aspects, wherein the processor detects an anatomical differentiation region that anatomically differentiates the dentition constituent tissue on the basis of the image data, and sets the anatomical differentiation region as the segmentable region.

Accordingly, the position of the detail observation image with respect to the anatomical differentiation region can be appropriately set.

A tenth aspect is the image processing apparatus according to the ninth aspect, wherein the processor sets a position in the anatomical differentiation region as the detail observation reference position.

As a result, the internal structure of the anatomical differentiation region can be observed using the detail observation image.

The processor may perform processing of differentiating a dentition region and an alveolar bone region on the basis of the three-dimensional image data, and perform image processing in which the dentition region and the alveolar bone region are displayed in different manners as the dentition developed image. As a result, the dentition region and the alveolar bone region are displayed in a differentiated manner in the dentition developed image, so that it is easy to grasp the position of the segmentable region.

The processor may cause the dentition region and the alveolar bone region to have different colors in the dentition developed image. As a result, it is easy to differentiate and recognize the dentition region and the alveolar bone region.

The processor may set transparency to the alveolar bone region to such an extent that the dentition region can be seen through. As a result, also for the portion of the dentition present inside the alveolar bone, it is possible to observe the segmentable region while grasping the positional relationship between the alveolar bone and the dentition.

The processor may indicate functional dentition constituent tissue, which is function-specific tissue constituting dentition constituent tissue of a living body, by a boundary surface between presence and absence, and perform line-of-sight direction-corresponding transparency image processing, which is image processing in which transparency changes depending on the line-of-sight direction, on the boundary surface. As a result, it is easy to visually recognize the shape of the functional dentition constituent tissue.

When the element increasing the transparency is the pro-transparency element, increasing the transparency is the pro-transparency, the element for decreasing the transparency is the anti-transparency element, and decreasing the transparency is the anti-transparency, the line-of-sight direction-corresponding transparency image processing may be a combination of at least any one of the following group A with at least any one of the following group B.
Group A:
   ▪ Processing of enlarging or increasing the pro-transparency element of the frontally-viewed boundary surface described above
   ▪ Processing of promoting pro-transparency of the frontally-viewed boundary surface described above
   ▪ Processing of downsizing, or reducing or eliminating the anti-transparency element of the frontally-viewed boundary surface described above
   ▪ Processing of suppressing anti-transparency of the frontally-viewed boundary surface described above
Group B:
   ▪ Processing of downsizing, or reducing or eliminating the pro-transparency element of the obliquely-viewed or laterally-viewed boundary surface described above
   ▪ Processing of suppressing pro-transparency of the obliquely-viewed or laterally-viewed boundary surface described above
   ▪ Processing of enlarging or increasing the anti-transparency element of the obliquely-viewed or laterally-viewed boundary surface described above
   ▪ Processing of promoting anti-transparency of the obliquely-viewed or laterally-viewed boundary surface described above

As a result, it is easy to visually recognize the three-dimensional shape of the functional dentition constituent tissue.

An eleventh aspect is the image processing apparatus according to any one of the first to tenth aspects, wherein the detail observation image includes three cross-sectional images orthogonal to each other.

In this case, since the detail observation image includes the three cross-sectional images orthogonal to each other, it is easy to observe the segmentable regions in detail.

A twelfth aspect is the image processing apparatus according to the eleventh aspect, wherein the processor identifies a type of the segmentable region, and the rule individually sets, by type, directions of the three cross-sectional images passing through the detail observation reference position.

As a result, the direction of the cross-sectional image can be individually set for each type.

A thirteenth aspect is the image processing apparatus according to any one of the first to twelfth aspects, further including a user interface, wherein the processor identifies a plurality of the segmentable regions, displays, on the display, the dentition developed image in which the plurality of segmentable regions are respectively displayed at a plurality of the segmentable region corresponding positions, receives designation of some of the plurality of segmentable regions through the user interface, and displays, on the display, the detail observation image corresponding to the some of the segmentable regions received.

As a result, it is possible to observe in detail the detail observation image corresponding to the some of the segmentable regions designated from the plurality of segmentable regions.

A fourteenth aspect is the image processing apparatus according to any one of the first to thirteenth aspects, further including a user interface, wherein the processor changes the detail observation reference position in response to a reference position change operation through the user interface.

As a result, by changing the reference position of the detail observation image, it is easy to observe the segmentable region at various positions.

A fifteenth aspect is an image processing method of processing image data obtained by computed tomography (CT) imaging of dentition constituent tissue to generate an image for diagnosis, the method including: generating three-dimensional image data of the dentition constituent tissue on the basis of the image data; identifying a segmentable region in the dentition constituent tissue on the basis of the image data; identifying a three-dimensional position at which the segmentable region is present in a coordinate system of the three-dimensional image; generating a dentition developed image in which the dentition constituent tissue is developed on the basis of the three-dimensional image data; identifying a segmentable region corresponding position corresponding to the three-dimensional position in the dentition developed image; displaying the dentition developed image in which the segmentable region is displayed at the segmentable region corresponding position; generating a detail observation image of the segmentable region from the three-dimensional image data on the basis of a correspondence between the segmentable region corresponding position and the three-dimensional position by designation of the segmentable region corresponding position in the dentition developed image; determining a detail observation reference position that is a reference position for generating the detail observation image according to a predetermined rule; and displaying the detail observation image.

According to this image processing method, the position of the detail observation image can be appropriately set in the dentition developed image.

A sixteenth aspect is a program for processing image data obtained by computed tomography (CT) imaging of dentition constituent tissue to generate an image for diagnosis, the program being configured to cause a computer to execute processing of: generating three-dimensional image data of the dentition constituent tissue on the basis of the image data; identifying a segmentable region in the dentition constituent tissue on the basis of the image data; identifying a three-dimensional position at which the segmentable region is present in a coordinate system of the three-dimensional image; generating a dentition developed image in which the dentition constituent tissue is developed on the basis of the three-dimensional image data; identifying a segmentable region corresponding position corresponding to the three-dimensional position in the dentition developed image; displaying the dentition developed image in which the segmentable region is displayed at the segmentable region corresponding position; generating a detail observation image of the segmentable region from the three-dimensional image data on the basis of a correspondence between the segmentable region corresponding position and the three-dimensional position by designation of the segmentable region corresponding position in the dentition developed image; determining a detail observation reference position that is a reference position for generating the detail observation image according to a predetermined rule; and displaying the detail observation image.

According to this program, the position of the detail observation image can be appropriately set in the dentition developed image.

### Appendices

Various aspects of the present disclosure will collectively be described below as appendices.

### Appendix 1

An image processing apparatus that processes image data obtained by computed tomography (CT) imaging of dentition constituent tissue to generate an image for diagnosis, the image processing apparatus including:
a storage configured to store the image data;
a processor; and
a display configured to display an observation image on a basis of output of the processor,
wherein the processor is configured to:
   generate three-dimensional image data of the dentition constituent tissue on a basis of the image data,
   identify a segmentable region in the dentition constituent tissue on a basis of the image data,
   identify a first position that is a three-dimensional position, at which the segmentable region is present, in a coordinate system of the three-dimensional image,
   generate a first image (dentition developed image), in which the dentition constituent tissue is developed, on a basis of the three-dimensional image data,
   identify a second position (segmentable region corresponding position), corresponding to the first position, in the first image,
   display, on the display, the first image, in which the segmentable region is displayed, at the second position,
   generate second image (detail observation image) which is a detail observation of the segmentable region from the three-dimensional image data on a basis of a correspondence between the second position and the first position by designation of the second position in the first image,
   determine a third position (detail observation reference position), that is a reference position for generating the second image, according to a predetermined rule, and
   display the second image on the display.

### Appendix 2

The image processing apparatus according to Appendix 1 or Appendix 2, wherein
the rule is a rule for determining the third position on a basis of position information of at least a part of the region.

### Appendix 3

The image processing apparatus according to Appendix 1 or Appendix 2, wherein
the processor is further configured to determine an observation direction of the second image on a basis of position information of at least a part of the segmentable region identified in the dentition constituent tissue.

### Appendix 4

The image processing apparatus according to Appendix 1 or Appendix 2, wherein
the third position includes a position in a buccolingual direction.

### Appendix 5

The image processing apparatus according to Appendix 1 or Appendix 2, wherein the processor is further configured to identify a type of the segmentable region, and
wherein the rule is individually preset for the type.

### Appendix 6

The image processing apparatus according to Appendix 1 or Appendix 2, wherein the processor is further configured to:
detect a biological feature region in the dentition constituent tissue on a basis of the image data, and
set the biological feature region as the segmentable region.

### Appendix 7

The image processing apparatus according to Appendix 6, wherein
the processor is further configured to display, on the display, a third image (biological feature region arrangement developed image) in which an annotation image, indicating the biological feature region, is superimposed on the first image such that the annotation image is located at the second position.

### Appendix 8

The image processing apparatus according to Appendix 6, wherein
the processor is further configured to set a center of the biological feature region as the third position.

### Appendix 9

The image processing apparatus according to Appendix 1 or Appendix 2, wherein
the processor is further configured to:
detect an anatomical differentiation region that anatomically differentiates the dentition constituent tissue on a basis of the image data, and
set the anatomical differentiation region as the segmentable region.

### Appendix 10

The image processing apparatus according to Appendix 9, wherein
the processor is further configured to set a position in the anatomical differentiation region as the third position.

### Appendix 11

The image processing apparatus according to Appendix 1 or Appendix 2, wherein
the second image includes three cross-sectional images orthogonal to each other.

### Appendix 12

The image processing apparatus according to Appendix 11, wherein
the processor is further configured to:
identify a type of the segmentable region, and
individually set, for the type, directions of the three cross-sectional images passing through the third position.

### Appendix 13

The image processing apparatus according to Appendix 1 or Appendix 2, further including a user interface, and
wherein the processor is further configured to
identify a plurality of segmentable regions,
display, on the display, the first image in which the plurality of segmentable regions are respectively displayed at a plurality of second positions that is a plurality of positions at which the plurality of segmentable regions are present,
receive designation of some of the plurality of segmentable regions through the user interface, and
display, on the display, the second image corresponding to the some of the plurality of segmentable regions.

### Appendix 14

The image processing apparatus according to Appendix 1 or Appendix 2, further including a user interface, and
wherein the processor is further configured to change the third position in response to a reference position change operation through the user interface.

### Appendix 15

A computer-executable image processing method for processing image data obtained by computed tomography (CT) imaging of dentition constituent tissue to generate an image for diagnosis, the method including:
generating, using a processor, three-dimensional image data of the dentition constituent tissue on a basis of the image data;
identifying, using the processor, a segmentable region in the dentition constituent tissue on a basis of the image data;
identifying, using the processor, a first position that is a three-dimensional position, at which the segmentable region is present, in a coordinate system of the three-dimensional image;
generating, using the processor, a first image, in which the dentition constituent tissue is developed, on a basis of the three-dimensional image data;
identifying, using the processor, a second position corresponding to the first position in the first image;
displaying, using the processor, the first image in which the segmentable region is displayed at the second position;
generating, using the processor, second image which is a detail observation of the segmentable region from the three-dimensional image data on a basis of a correspondence between the second position and the first position by designation of the second position in the first image;
determining a third position, that is a reference position for generating the second image, according to a predetermined rule; and
displaying the second image.

### Appendix 16

A non-transitory computer-readable storage medium storing instructions for processing image data obtained by computed tomography (CT) imaging of dentition constituent tissue to generate an image for diagnosis that when executed by a processor causes the processor to implement a method including:
generating three-dimensional image data of the dentition constituent tissue on a basis of the image data;
identifying a segmentable region in the dentition constituent tissue on a basis of the image data;
identifying a first position that is a three-dimensional position, at which the segmentable region is present, in a coordinate system of the three-dimensional image;
generating a first image, in which the dentition constituent tissue is developed, on a basis of the three-dimensional image data;
identifying a second position corresponding to the first position in the first image;
displaying the first image in which the segmentable region is displayed at the second position;
generating second image which is a detail observation of the segmentable region from the three-dimensional image data on a basis of a correspondence between the second position and the first position by designation of the second position in the first image;
determining a third position that is a reference position for generating the second image according to a predetermined rule; and
displaying the second image.

Although the present invention has been described in detail as above, the above description is illustrative in all phases, and the present invention is not limited thereto. It is understood that numerous modifications not illustrated can be assumed without departing from the scope of the present invention.

While the disclosure has been shown and described in detail, the foregoing description is in all aspects illustrative and not restrictive. It is therefore understood that numerous modifications and variations can be devised.

## Claims

1. An image processing apparatus that processes image data obtained by computed tomography imaging of dentition constituent tissue to generate an image for diagnosis, the image processing apparatus comprising:
a storage that stores said image data;
a processor; and
a display that displays an observation image on the basis of output of said processor,
wherein said processor
generates three-dimensional image data of said dentition constituent tissue on the basis of said image data,
identifies a segmentable region in said dentition constituent tissue on the basis of said image data,
identifies a three-dimensional position at which said segmentable region is present in a coordinate system of said three-dimensional image,
generates a dentition developed image in which said dentition constituent tissue is developed on the basis of said three-dimensional image data,
identifies a segmentable region corresponding position corresponding to said three-dimensional position in said dentition developed image,
displays, on said display, said dentition developed image in which said segmentable region is displayed at said segmentable region corresponding position,
generates a detail observation image of said segmentable region from said three-dimensional image data on the basis of a correspondence between said segmentable region corresponding position and said three-dimensional position by designation of said segmentable region corresponding position in said dentition developed image,
determines a detail observation reference position that is a reference position for generating said detail observation image according to a predetermined rule, and
displays said detail observation image on said display.

2. The image processing apparatus according to claim 1, wherein
said rule is a rule for determining said detail observation reference position on the basis of position information of at least a part of said segmentable region.

3. The image processing apparatus according to claim 1 or claim 2, wherein
said processor determines an observation direction of said detail observation image on the basis of position information of at least a part of said segmentable region identified in said dentition constituent tissue.

4. The image processing apparatus according to claim 1 or claim 2, wherein
said detail observation reference position includes a position in a buccolingual direction.

5. The image processing apparatus according to claim 1 or claim 2, wherein
said processor
identifies a type of said segmentable region, and
said rule is individually preset for said type.

6. The image processing apparatus according to claim 1 or claim 2, wherein
said processor
detects a biological feature region in said dentition constituent tissue on the basis of said image data, and
sets said biological feature region as said segmentable region.

7. The image processing apparatus according to claim 6, wherein
said processor displays, on said display, a biological feature region arrangement developed image in which an annotation image indicating said biological feature region is superimposed on said dentition developed image such that said annotation image is located at said segmentable region corresponding position.

8. The image processing apparatus according to claim 6, wherein
said processor sets a center of said biological feature region as said detail observation reference position.

9. The image processing apparatus according to claim 1 or claim 2, wherein
said processor
detects an anatomical differentiation region that anatomically differentiates said dentition constituent tissue on the basis of said image data, and
sets said anatomical differentiation region as said segmentable region.

10. The image processing apparatus according to claim 9, wherein
said processor sets a position in said anatomical differentiation region as said detail observation reference position.

11. The image processing apparatus according to claim 1 or claim 2, wherein
said detail observation image includes three cross-sectional images orthogonal to each other.

12. The image processing apparatus according to claim 11, wherein
said processor
identifies a type of said segmentable region, and
said rule individually sets, for said type, directions of said three cross-sectional images passing through said detail observation reference position.

13. The image processing apparatus according to claim 1 or claim 2, further comprising a user interface,
wherein said processor
identifies a plurality of said segmentable regions,
displays, on said display, said dentition developed image in which said plurality of segmentable regions are respectively displayed at a plurality of said segmentable region corresponding positions,
receives designation of some of said plurality of segmentable regions through said user interface, and
displays, on said display, said detail observation image corresponding to said some of the segmentable regions received.

14. The image processing apparatus according to claim 1 or claim 2, further comprising a user interface,
wherein said processor changes said detail observation reference position in response to a reference position change operation through said user interface.

15. An image processing method of processing image data obtained by computed tomography imaging of dentition constituent tissue to generate an image for diagnosis, the method comprising:
generating three-dimensional image data of said dentition constituent tissue on the basis of said image data;
identifying a segmentable region in said dentition constituent tissue on the basis of said image data;
identifying a three-dimensional position at which said segmentable region is present in a coordinate system of said three-dimensional image;
generating a dentition developed image in which said dentition constituent tissue is developed on the basis of said three-dimensional image data;
identifying a segmentable region corresponding position corresponding to said three-dimensional position in said dentition developed image;
displaying said dentition developed image in which said segmentable region is displayed at said segmentable region corresponding position;
generating a detail observation image of said segmentable region from said three-dimensional image data on the basis of a correspondence between said segmentable region corresponding position and said three-dimensional position by designation of said segmentable region corresponding position in said dentition developed image;
determining a detail observation reference position that is a reference position for generating said detail observation image according to a predetermined rule; and
displaying said detail observation image.

16. A program for processing image data obtained by computed tomography imaging of dentition constituent tissue to generate an image for diagnosis, the program being configured to cause a computer to execute processing of:
generating three-dimensional image data of said dentition constituent tissue on the basis of said image data;
identifying a segmentable region in said dentition constituent tissue on the basis of said image data;
identifying a three-dimensional position at which said segmentable region is present in a coordinate system of said three-dimensional image;
generating a dentition developed image in which said dentition constituent tissue is developed on the basis of said three-dimensional image data;
identifying a segmentable region corresponding position corresponding to said three-dimensional position in said dentition developed image;
displaying said dentition developed image in which said segmentable region is displayed at said segmentable region corresponding position;
generating a detail observation image of said segmentable region from said three-dimensional image data on the basis of a correspondence between said segmentable region corresponding position and said three-dimensional position by designation of said segmentable region corresponding position in said dentition developed image;
determining a detail observation reference position that is a reference position for generating said detail observation image according to a predetermined rule; and
displaying said detail observation image.
